# EUROPEAN PATENT APPLICATION

(11) **EP 3 736 576 A1**
(43) Date of publication of application: **11.11.2020**
(21) Application number: 20182892.8
(22) Date of filing: 06.11.2018
(51) Int. Cl.: G01N 33/94, G01N 33/50

(54) **THERAPEUTIC SUBSTANCES, THEIR PREPARATION AND DIAGNOSTIC PROCEDURE**

(30) Priority: 06.11.2017 GB 201718319; 06.11.2017 GB 201718320; 06.11.2017 US 201762582025 P; 23.03.2018 GB 201804737
(62) Divisional of application: 18815791.1
(71) Applicant: King's College London, London WC2R 2LS (GB)
(72) Inventor: DAZZI, Francesco, London, WC2R 2LS (GB)
(74) Representative: Script IP Limited

(57) **Abstract**

The invention relates to apoptotic MSCs for use in the treatment of immune-mediated disease or conditions or in regenerative medicine to stimulate tissue repair. The invention also relates to methods and uses and compositions. The invention also relates to a method of identification of suitably potent MSCs that can best be induced to undergo apoptosis and/or to produce PGE2, comprising contacting the live MSCs in vitro with a biological sample from a patient, and detecting either the presence of apoptotic MSCs among the live MSCs in vitro or elevated levels of prostaglandin E2 (PGE2) in the sample, wherein the presence of apoptotic MSCs among the live MSCs or elevated levels of PGE2 in vitro is indicative of potency.

## Description

### TECHNICAL FIELD

The present invention relates to a method for identifying patients able to respond to immunosuppressive treatment using live mesenchymal stromal cells (MSCs), and for therapeutic options available to responding and non-responding patients and for methods for preparing agents for use in the methods.

### BACKGROUND

Inflammatory disorders impart a massive burden on health services worldwide. Although biologics have produced a positive impact, they are not curative and are associated with substantial toxicity. Cell-based immunomodulation is an emerging opportunity supported by the encouraging results obtained in the clinical setting. Mesenchymal stromal cells (MSC) are probably amongst the most promising. Several studies have shown that MSC control inflammation and, as a consequence, regulate tissue homeostasis by promoting residual host tissue repair activity. MSC mediated immunosuppression virtually affects any types of immune cells and does not require any histocompatibility with the target cells to be produced. Such properties and their versatility have therefore attracted much interest for the opportunity of harnessing MSC for the treatment of immune mediated disorders. Proof of concept already exists that MSC have a beneficial effect in graft-versus-host disease (GvHD). GvHD is a potentially lethal complication that affects approximately 40% of patients undergoing allogeneic haemopoietic stem cell transplantation (HSCT). When the disease does not respond to steroids - this happens in about 10-20% of the cases - patients usually die. Early phase II studies have reported that about half of patients with severe steroid refractory GvHD experience a clinical response following MSC infusions. There is also initial evidence that MSC therapies could be successfully used to control rejection of transplanted organs, improve autoimmune disorders like multiple sclerosis, Crohn's disease, some forms of arthritis and severe acute inflammatory conditions like acute respiratory distress syndrome.

However, the clinical results described are variable and proof of efficacy inconclusive. This has been attributed to the lack of a potency assay capable of providing criteria to select the most effective cell preparation, but the limited understanding of the mechanisms underpinning the MSC therapeutic effects has thus far prevented any progress in this direction. The other side of the coin is patient selection. The data available - strongly backed by pre-clinical studies - indicate that choosing the appropriate timing for MSC infusion is fundamental for clinical efficacy, largely irrespective of the cell preparation. Timing does not seem necessarily related to disease severity but rather to the inflammatory features of the disease. In fact, MSC are not constitutively immunosuppressive, but they do so only after being exposed to an inflammatory environment that 'licenses' their properties. The pattern of molecular cues that triggers MSC therapeutic activity in disease remains an unanswered question which, if properly addressed, have the potential to identify which patients are more likely to benefit from MSC therapies. The only evidence to date from animal models and *in vitro* systems have implicated a role for interferon-γ (IFN-γ), tumour necrosis factor-α (TNF-α) and interleukin-1β (IL-1β) in MSC 'licensing' but none of these molecules have been confirmed in the clinical setting. Therefore, probably the most urgent unmet need in MSC therapeutics is understanding which patients are more likely to respond to the treatment. Identifying the signature associated with MSC-responders would allow to inform patient stratification, design highly powered clinical trials, and eventually maximise the already promising MSC-based therapeutic strategies.

One of the most puzzling observations in the field of MSC therapeutics is that most of the cells infused reside transiently in the lungs almost immediately after infusion, before becoming undetectable within a few hours. Current knowledge cannot provide an explanation for this paradox and so a better understanding of the mechanisms underlying MSC therapeutic activity would be desirable in order to maximise its utility.

The applicants have made significant progress in this respect, so that patient selection can be made and therapeutic options for all patients can be identified and broadened.

### SUMMARY OF THE INVENTION

The applicants have observed that if MSC are injected into recipients who harbour activated immune cells with cytotoxic functions, they rapidly undergo apoptotic cell death. This cell death does not occur in healthy recipients or in recipients in which the inflammation does not contain cytotoxic cells. Paradoxically, the therapeutic effect can only be delivered in recipients that contain cytotoxic cells and kill the infused MSC. The mechanism that account for this apparent paradox is that it is specifically apoptotic MSCs that have the ability to trigger in the recipient an immunosuppressive activity which is largely effected by indoleamine deoxidase (IDO).

This finding therefore provides a clear basis for patient stratification.

According to a first aspect of the present invention there is provided a method for identifying a patient likely to respond to immunosuppressive treatment using live mesenchymal stromal cells (MSCs), said method comprising determining whether a sample from said patient is able to induce at least some apoptosis to occur in live MSCs *in vitro,* and where the ability of the sample to induce said apoptosis is indicative of responsiveness of said patient to said immunosuppressive treatment.

Suitable patient samples for use in the method of the invention are samples containing immune cells such as peripheral blood mononuclear cells. Thus, suitable samples are blood or serum samples, but other samples such as biopsy samples or cerebrospinal fluid (CSF) samples may be used. If required, samples may be plated before use to concentrate immune cells.

In a particular embodiment of the method of the first aspect of the invention, the ability of the sample to cause apoptosis in MSCs is determined by incubating said sample with live MSCs and thereafter, detecting the presence of apoptotic MSCs in the incubate. Suitably, where at least 11%, optionally 12.5% of the MSCs added become apoptotic after the incubation, the patients would be regarded as being "responsive" to treatment using live MSCs. In a particular embodiment, the threshold of MSC apoptosis indicative of a "responsive" host is 11.5%, or 12.5%, or 13.5%.

The number of patient's cells such as PBMCs used in the assay will vary depending upon factors such as the sample size, and the method of detection. However, typically the ratio of immune cells : MSCs mixed together for incubation will be in the range of from 1:1 to 60:1 for example from 2:1 to 40:1. These MSCs will suitably be from any healthy donor or source.

Incubation is suitably carried out for a period sufficient to allow valid results to be determined, but rapidly enough to allow therapeutic options to be explored quickly, even in the case of severe disease conditions. Typically, this will be in the range of from 1-24 hours, or 2-24 hours, for example from 1-6 hours, or 1-5 hours, or 2-6 hours, and in particular from 3-5 hours, such as about 4 hours.

The presence and amount of apoptotic MSCs in the incubate may be determined by any suitable assay or method. Such methods may include confocal microscopy, flow cytometry, mass cytometry, other antibody-based detection methods, DNA amplification and/or detection such as polymerase chain reactions, immunoassay methods such as ELISA or immunoenzymatic assays or metabolic assays. Suitable labelling of apoptotic cells to allow detection using such methods may be based upon annexin-V binding or caspase activity. Microscopy methods may include stains, for example stains for mitochondria or DNA may detect respectively early stage apoptosis, where active mitochondria are disrupted, or later stage apoptosis when DNA fragmentation occurs.

However, the applicants have now identified that prostaglandin E2 (PGE2) is a molecular marker that is induced by patients' PBMC after physical contact with MSC and that this activity correlates with the ability of PBMC to induce MSC apoptosis. In particular, it appears that MSCs are induced to secrete high levels of PGE2 on contact with patients' PBMC in a caspase dependent manner. As a result, this provides a convenient method for detecting the ability of a patient to induce apoptosis in MSCs in vitro, as the level of PGE2 can be measured more simply and objectively by an independent operator for example, using a commercially available ELISA kit.

Thus in a particular embodiment of the invention, the ability of the sample to cause apoptosis in MSCs is determined by incubating said sample with live MSCs, detecting the level of prostaglandin E2 (PGE2) in the incubate or a supernatant obtained therefrom, and relating the level to the responsiveness of said patient to said immunosuppressive treatment. As illustrated hereinafter, levels of PGE2 in a supernatant from an incubate in excess of 4000pg/ml is generally indicative of responsiveness of said patient to immunosuppressive treatment using live MSCs. These levels may be determined using any conventional method, such as immunoassay methods, but a particularly convenient method involves the use of a quantitative ELISA. A variety of kits are available commercially for this purpose.

By using the method of the invention, patients most likely to respond to MSC therapy can be identified. As a result, these patients may be prescribed the most appropriate treatment, with the realistic expectation that the therapy will be more successful.

The method of the invention provides a highly sensitive test which can accurately identify 'responders' and 'non-responders' to MSC therapy. It has been shown to achieve 100% sensitivity in trials and 92% sensitivity in a clinical study. Furthermore, the test has a high specificity, as a clinical response has been found in 90% of patients treated.

The method provides a means of using MSCs *in vitro.* Thus, in a second aspect, the invention provides a method of using live mesenchymal stromal cells (MSCs) *in vitro,* the method comprising contacting the live MSCs *in vitro* with a biological sample from a patient, and either detecting the presence of apoptotic MSCs among the live MSCs *in vitro,* or detecting elevated levels of PGE2 in the mixture, wherein the presence of apoptotic MSCs among the live MSCs *in vitro* or of elevated levels of PGE2 is indicative of responsiveness of the patient to an immunosuppressive treatment using live MSCs.

The method of the second aspect is suitably carried out using a plurality of samples of live MSCs *in vitro,* each of which is contacted with biological samples from different patients in a plurality of patients, and the presence of apoptotic MSCs, or of elevated levels of PGE2, in each sample of live MSCs *in vitro* is detected. In this way, responders and non-responders may be identified, and suitably potent MSCs.

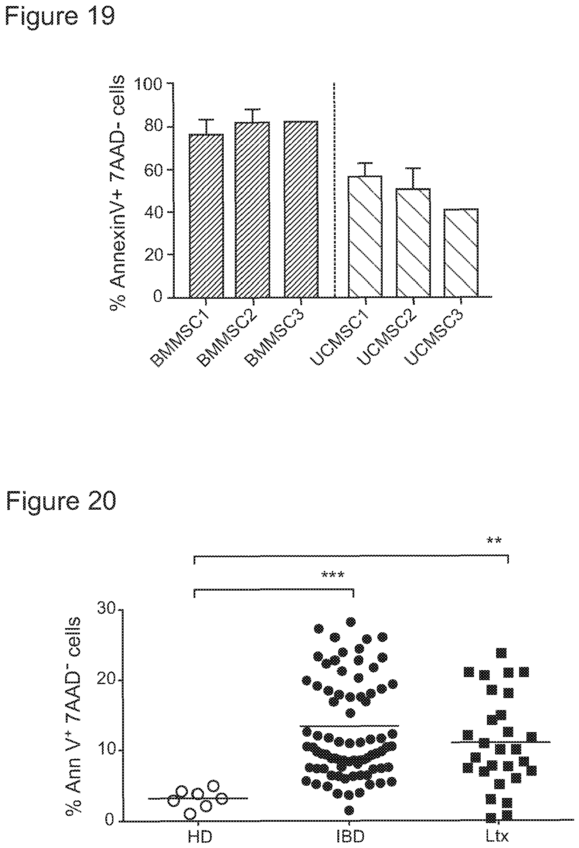

The method of the second aspect of the invention therefore also enables the identification of suitably potent MSCs that can best be induced to undergo apoptosis and/or to produce PGE2, thereby allowing the method of the invention to also be used as a potency assay. A potency assay is particularly advantageous since it has been shown that MSCs from different sources exhibit varying sensitivity to undergo apoptosis (see Figure 19). Furthermore, obtaining a plurality of samples or batches of MSCs of differing potency can assist in quality control and allow for the determining of consistency across batches.

In each instance, the contacting step suitably comprises incubating the biological sample with live MSCs and the detecting step suitably comprises detecting the presence of apoptotic MSCs in the incubate, or detecting elevated levels of PGE2 in the supernatant for the incubate. Suitable samples, detection levels etc. are similar to those used in the first aspect of the invention.

As set out below, the applicants have investigated the mechanisms underlying MSC activity using a Graft-versus-Host Disease (GvHD) model because there is proof of principle that MSC are efficacious in this case. It is demonstrated that the activated cytotoxic cells harboured in GvHD mice rapidly induce extensive *in vivo* caspase activation in infused MSC. The presence of activated cytotoxic cells in MSC recipients is required for inducing MSC apoptosis and, as a consequence, for triggering the MSC immunosuppressive effect.

These findings do not only explain the rapid clearance of infused MSC but also mechanistically reconcile their disappearance with their immunomodulatory activity. The link between cytotoxic cell activity, MSC apoptosis and immunosuppression is confirmed by the correlation in GvHD patients between high levels of cytotoxic activity against MSC and clinical responses. Recipient-mediated killing of MSC can therefore be regarded as the first mechanism-based biomarker for patient stratification.

Strikingly, as set out below, they have also found that apoptotic MSC generated ex *vivo* exhibit a potent immunosuppressive effect that is dependent on phagocyte-derived indoleamine 2,3-dioxygenase (IDO) activity and bypasses the need for cytotoxic cells in the recipient. As a result, patients who are not responsive the MSCs may be treated instead with apoptotic MSC.

Furthermore, this finding provides a means of confirming patient response after treatment with MSC or apoptotic MSC as appropriate. In particular, patient response can be gauged by analysing samples such as blood or serum samples for the presence of phagocytes and/or IDO, which act as biomarkers to indicate whether or not the treatment is working.

Suitable assays for phagocytosis are known in the art. For instance, phagocytosis may be assayed by measuring the engulfment of a cell substrate, such as erythrocytes that have be opsonized using serum or IgG, yeast particles such as zymosan or other particles such as metal beads. Samples are incubated with the substrate to allow engulfment to take place. After this, phagocytes are imaged using cytofluorimetric assays after antibody staining for phagocytes (for example anti-CD14 or anti-CD16) and the presence of the substrate/particle inside the cytoplasm detected. Alternatively, a functional assay may be carried out in which any free substrate is removed, and phagocytes the sample are lysed to release the engulfed substrate for detection.

Alternatively, IDO may act as a convenient biomarker for efficacy of treatment. This may be determined using various conventional methods including detection of RNA, IDO protein detection, for example using immunoassay techniques like ELISA, or by assaying for enzyme activity, as described for example by Braun et al. Blood, 2005, 106(7): 2375-2381. A further method involves the biochemical quantitation of the kynurenine pathway metabolites by chromatography. IDO activity in vivo can also be imaged at positron emission tomography with tryptophan tracers, as described by Bosnyak et al, 2015, Neuro Oncol 17(9): 1284-1292.

Thus, a patient determined to be responsive to immunosuppressive treatment using live MSCs using the method of the first or second aspects of the invention may be treated with MSCs. In addition however, a patient determined to be non-responsive to said immunosuppressive treatment may be treated with apoptotic MSCs. Thereafter, a patient's response to the treatment may be monitored by detecting phagocytes or IDO as described above.

A third aspect of the invention therefore provides a method of stratifying patients for treatment with MSC immunosuppression therapy, said method comprising carrying out a method according to the first and/or second aspects of the invention, identifying patients likely to respond to immunosuppressive treatment using live mesenchymal stromal cells (MSCs) for treatment with live MSCs, and identifying those who are not likely to respond to said immunosuppressive treatment for treatment with apoptotic MSCs.

The method of the first and/or second aspect of the invention may also be used to stratify other types of patients or subjects, for example, injured patients or patients suffering from other inflammatory conditions. Figure 20 shows the presence of cytotoxic cells against stromal cells in other inflammatory conditions.

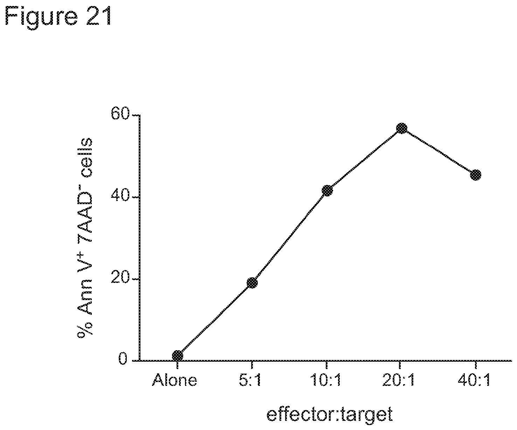

The methods of the invention can also be used as an assay to quantify the ability of a patient to control inflammation independently of MSC infusions. Figure 21 shows that fibroblasts, a cell type affiliated to MSC, from the skin can also be killed by activated cytotoxic cells. This suggests that the formation of cytotoxic cells in a tissue, a frequent event after injury, may induce stromal cells to undergo apoptosis and initiate the anti-inflammatory activity and, as a consequence, tissue repair. Given therefore that cytotoxic cells produced following injury may induce apoptosis and initiate the immunomodulation required to promote tissue regeneration, detection of the biomarker according to the methods of the invention can therefore be used to detect fitness to regenerate.

According to the present invention there is therefore provided a method for identifying a subject's fitness to recover from tissue injury, said method comprising determining whether a sample from said subject is able to induce at least some apoptosis to occur in live MSCs *in vitro,* and/or detecting elevated levels of PGE2, and wherein the ability of the sample to induce said apoptosis, or the presence of elevated levels of PGE2, is indicative of said patient's capacity to recover.

The capacity to recover from tissue injury may be quantifiable by detecting the presence and amount of apoptotic MSCs and/or detecting elevated levels of PGE2 in the incubate according to any suitable assay or method described hereinabove.

By using the method of the invention, subjects most likely to recover from tissue injury can be identified, i.e. those with relatively high levels of MSC killer cells. Conversely, subjects with a reduced likelihood of recovery from tissue injury can be identified and prescribed the most appropriate treatment to promote tissue recovery.

Apototic MSCs (ApoMSC) have been used previously for the treatment of diseases such as myocardial infarction (Thum et al. 2005 JACC Volume 46, Issue 10, 15 November 2005, Pages 1799-1802) or sepsis or sepsis induced injury of lung, kidney or liver (Chang et al., J Transl Med 2012, 10:244; Chang et al, Am J Transl Res 2014, 6:439; Chen et al, J Pineal Res 2014, 57:16) The cells are often combined with melatonin and may show effects which are better than live MSCs in these circumstances. However, there has been no prior indication that effects would be different in different patient populations.

The production of apoptotic MSCs is a relatively complex procedure and requires additional complexity in the production and storage, making them a less appealing product than MSCs. A particular advantage of using the stratification method of the third aspect of the invention is that those patients who will benefit most from ApoMSC therapy in preference to MSC therapy can be identified.

Furthermore, by determining the mechanism by which MSCs operate and by identifying the need for apoptosis and the benefits the use of ApoMSCs in the treatment of immune disease, the applicants have identified that these cells may be used in the treatment of a wide range of immune diseases. As mentioned above, apoptotic cells have been indicated as being potentially useful in sepsis. This is a disease which, although it may have an immune component, is characterised by an overpowering uncontrolled inflammatory response to an external stimulus such as an infection. By understanding the mechanism of action of MSCs, the applicants have allowed the range of disease treatable using ApoMSCs to be expanded to include allo-immune disease (like GvhD or solid organ transplantation), autoimmune diseases, which are (diseases whose principle cause is an inherent problem with the host immune system. In addition, by identifying the mechanism, it can be deduced that ApoMSCs could have effects on overpowering uncontrolled inflammatory conditions other than sepsis, including acute respiratory distress syndrome.

The applicants have, as a result of this work, found that apoptotic MSCs are useful in the treatment of the diseases or conditions listed above. These will include Graft-vs-Host-Disease, to prevent or treat rejection of transplanted organs such as transplanted liver or kidney or heart; as well as autoimmune diseases like multiple sclerosis and other neurodegenerative disorders, inflammatory bowel diseases (Crohn's disease and ulcerative colitis), some forms of arthritis (like rheumatoid arthritis, ankylosing spondylitis, psoriatic arthritis), and acute respiratory distress syndrome. Additionally, apoptotic MSC can produce therapeutic benefit in regenerative medicine by stimulating tissue repair.

In this case, patient stratification may not be necessary, as the presence of ApoMSCs may have a similar effect on responding patients, as non-responding patients. However, it is also possible that the use of live MSCs has advantages in responder patients, and that stratification would still be beneficial.

Furthermore, ApoMSCs have previously been prepared by methods including the use of hypoxia or the use of serum-free media for culture, or by the use of hydrogen peroxide. These conditions result in cells of poor and/or heterogenous quality including necrotic cells, which would mean that they may not be acceptable for clinical application. In particular, the presence of necrotic cells is likely to promote inflammation rather than inhibiting it and so these cells could exacerbate rather than treat immune diseases.

WO2017/051421 describes a method in which the propagation of live MSCs is enhanced by addition of low levels of apoptosis inducing agents, which are added to cultures over an extended period of time of at least 3 days. Although the compositions obtained may contain apoptotic cells, these were found to be 'floating' cells and not part of the target colonies. The treatment increased the number of live MSCs and/or modified the size of the colony forming units obtained.

The applicants have developed a method by which ApoMSCs can be prepared in a manner which allows them to be used therapeutically.

In particular, apoptotic MSCs are produced for use in immunosuppression therapy by a method comprising incubating live MSCs with a pharmaceutically acceptable apoptosis inducing agent for a period sufficient to form a cell culture in which at least 30% of the MSCs are apoptotic within a period of less than 24 hours. In a particular embodiment, the method causes at least 50%, for example at least 60%, 70%, or 80% of the MSCs present in the cell culture to become apoptotic.

As used herein, the expression 'cell culture' refers to a growing culture such as may be found on a conventional culture plate or well.

Agents which induce apoptosis in cells are known in the art, and include chemical and biological agents. Pharmaceutically acceptable agents in accordance with the fourth aspect of the invention will allow the preparation to be used directly in clinical or pharmaceutical applications. In a particular embodiment, the pharmaceutically acceptable apoptosis inducing agent is a pharmaceutically acceptable biological agent. Examples of such agents may include serine proteases and/or anti-FAS antibodies, as well as psoralen which may induce apoptosis in conjunction with UV. A particularly useful serine protease is human Granzyme B (GrB), and this is suitably used in combination with GrB and anti-Fas antibody. Other agents include tumour necrosis-factor-α (TNF-α), TNF-related apoptosis-inducing ligand (TRAIL) and any other molecules used by the immune system to induce apoptosis in the target cells.

The apoptosis inducing agent(s) are suitably incubated with live MSCs for a period sufficient to induce the desired level of apoptosis. This will vary depending upon factors such as the nature of the agent used and the volume of the cells, but will typically be in the range of from 15 minutes to 25 hours, for example about 18 hours. After this time, cells become necrotic and therefore will no longer be suitable for therapeutic application in the treatment of immune disease for the reasons discussed above. This means also that ApoMSCs are suitably used within 24 hours of their production, and suitably within one hour, in particular, directly or immediately after production.

The concentration of the agent will also vary depending upon similar factors, but concentrations of from 1ng/ml to 50µg/ml, for example from 5-20µg/ml such as about 10µg/ml may be suitable in most cases. Where the agent used is an anti-Fas antibody or (FASL), in some embodiments, the concentration will be in excess of 100ng/ml.

The compositions comprising apoptotic MSCs obtainable using this method are useful in therapy and in particular in immunosuppression therapy.

In this context, pharmaceutical compositions comprising apoptotic MSCs as described above, in combination with a pharmaceutically acceptable carrier or excipient may be provided. Suitable carriers would be well known in the art, and include liquid carriers such as water or saline. The particular carrier will depend upon the mode of administration, but typically this will be by injection or infusion, for example by intravenous (i.v.), intraperitoneal (i.p), intramuscular (i.m.), intra-organ or subcutaneous (s.c) injection or infusion. In a particular embodiment, the composition will be administered intraperitoneally (i.p.) or subcutaneous (s.c) or by intravenous (i.v.).

The compositions may further contain some live MSCs. These live cells will contribute to the therapeutic effects in the case of 'responder' patients, but may be less useful or ineffective in the case of non-responder patients. However, they will not be harmful, and their presence will avoid the need for separating apoptotic cells from live MSCs prior to administration.

The MSCs in the composition may be genetically engineered to incorporate a suicide gene to better control their *in vivo* apoptosis. This provides a selective approach by which, independently of the presence of MSC killers in the patient, engineered MSC will be induced to undergo apoptosis *in vivo* only when they will have trafficked to the site of inflammation to which they are naturally chemo-attracted. In comparison to the anti-FasL approach, such an approach has the advantage that apoptosis can be executed *in vivo* selectively at the site of tissue injury and inflammation.

Therefore, according to the present invention, there is provided a composition comprising apoptotic MSCs and/or live MSCs as described above, which MSCs comprise a suicide mechanism. The suicide mechanism may comprise a suicide gene, particularly a caspase-inducible gene, such as caspase 9 or caspase 3.

Compositions may further comprise elements of culture media, used to support MSC growth provided these are pharmaceutically acceptable. Such media are known in the art, and are exemplified hereinafter.

In a further aspect, the invention provides a method for producing an immunosuppressive effect in a patient in need thereof, comprising administering to said patient an effective amount of apoptotic MSCs. The method may include as a preliminary step, determining whether said patient is likely to respond to immunosuppressive treatment using live mesenchymal stromal cells (MSCs), by detecting whether a sample from said patient is able to induce at least some apoptosis to occur in live MSCs *in vitro,* and where the sample is so able, administering to said patient an effective amount of live MSCs, and where said sample does not induce said apoptosis, administering to said patient an effective amount of apoptotic MSCs.

Conditions or diseases where immunosuppression may be required and which may be treated using this method include the diseases and conditions set out above. These will include Graft-vs-Host-Disease, prevention or treatment of rejection of transplanted organs such as transplanted liver or kidney or heart as well as autoimmune diseases like multiple sclerosis and other neurodegenerative disorders, inflammatory bowel diseases (Crohn's disease and ulcerative colitis), some forms of arthritis (like rheumatoid arthritis, ankylosing spondylitis, psoriatic arthritis. Lastly, apoptotic MSC can produce therapeutic benefit in regenerative medicine by stimulating tissue repair.

Also, as discussed above, the efficacy of the treatment in the patient may be monitored by detecting the presence of phagocytes or IDO in a sample, such as a blood or serum sample, taken from the patient after administration of MSCs, for example after a period of from 1 hour to 3 days post administration of MSCs or ApoMScs.

If required, kits may be provided to carry out one or more of the above methods, and these may, in some instances, form further aspect of the invention. Kits may comprise for example, means for detecting apoptotic MSCs, such as dye or stains, or labels as described above, and/or means for forming apoptotic MSCs in a pharmaceutically acceptable manner, such as the biological agents described above, and/or means for assaying for phagocytes or IDO in the patient monitoring step.

In summary therefore, the applicants have been able to shed light on the controversial topic of MSC therapeutics by identifying a crucial mechanism that explains several unresolved issues in the field. The first striking piece of information provides the resolution to the until now paradox that MSC are therapeutically efficacious despite the lack of engraftment. The applicants have demonstrated that MSC undergo extensive caspase activation and apoptosis after infusion in the presence of cytotoxic cells, and that this is a requirement for their immunosuppressive function. Although other recipient dependent reactions have been described as mediating MSC lysis *in vitro* and MSC clearance *in vivo,* this is the first indication of the instrumental role of *in vivo* MSC apoptosis in delivering immunosuppression after infusion. Furthermore, although several studies have reported the ability of apoptotic cells to modulate immune responses, the applicants have shown that *in vivo* naturally occurring cell death drives immunosuppression.

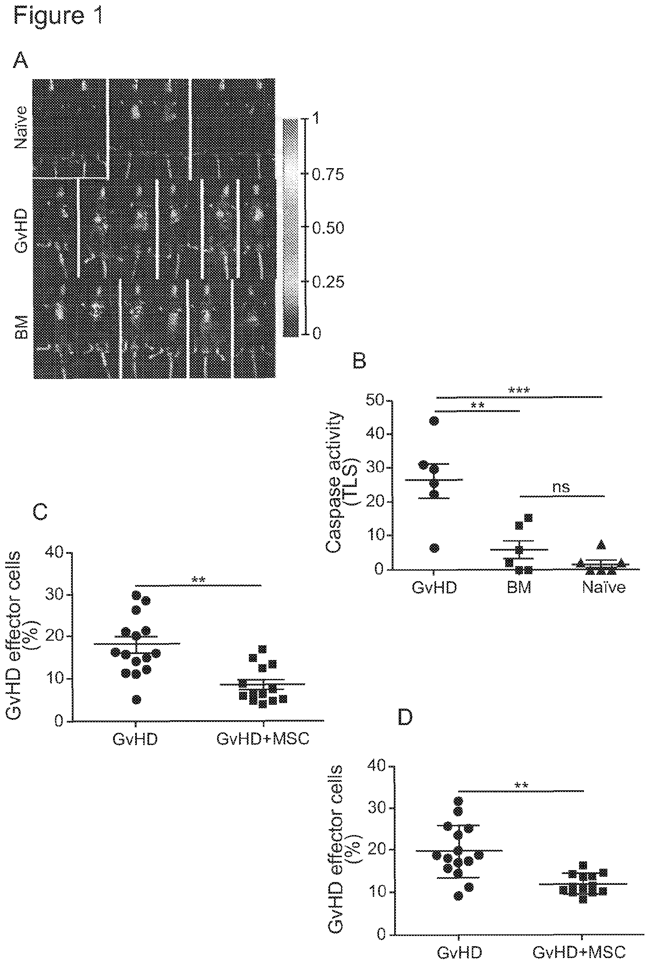

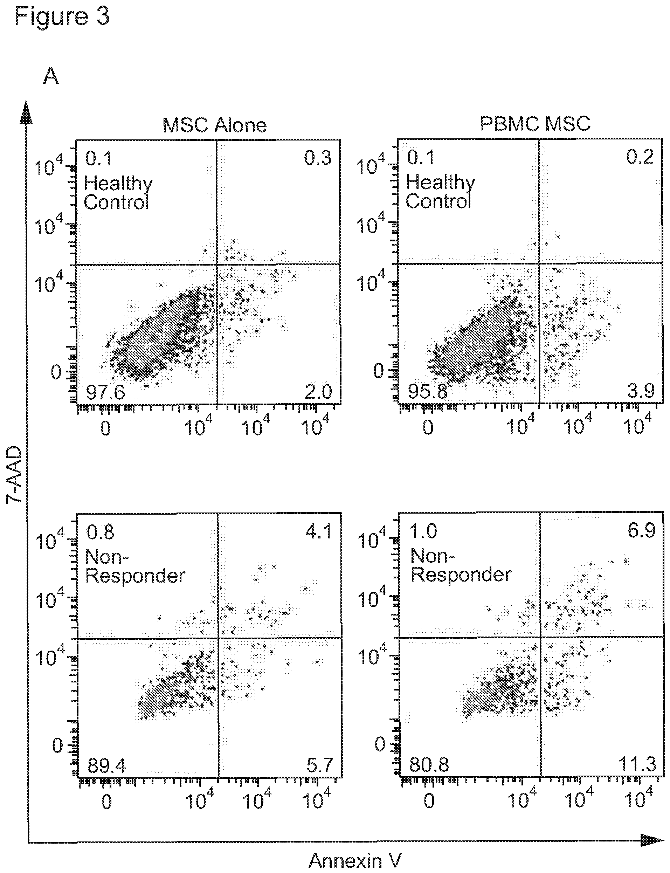

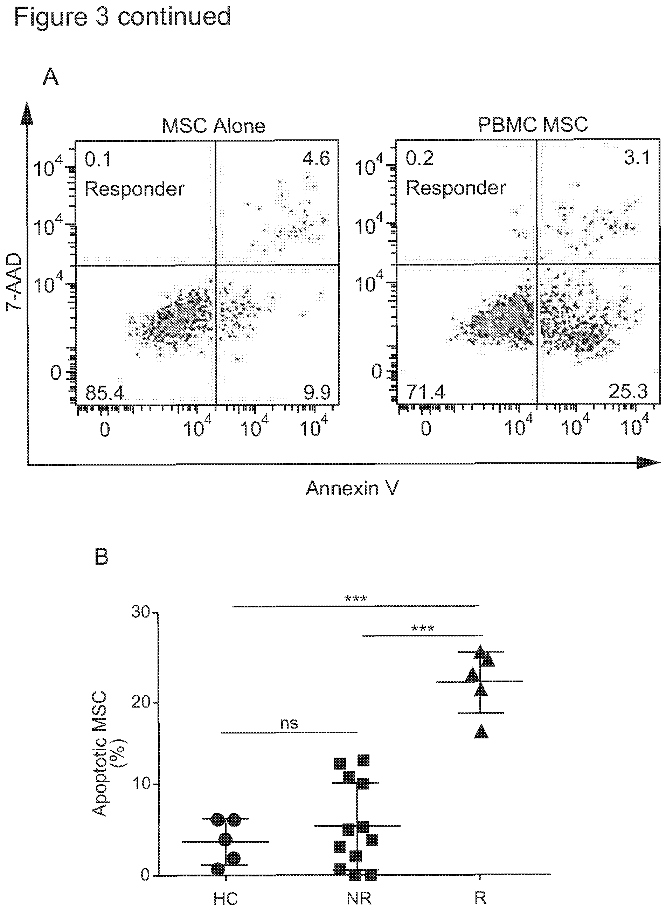

MSC apoptosis requires and is effected by cytotoxic granules contained in recipient cytotoxic cells that also mediate GvHD in recipient mice (Fig. 1, A and B, Fig. 2, C and D hereinafter). Importantly, the cytotoxic activity against MSC can also be detected in the PBMC of GvHD patients and it is predictive of clinical responses. Patients displaying high cytotoxicity respond to MSC, whilst those with low or absent cytotoxic activity do not improve following MSC infusion (Fig. 3, A and B). Therefore, the ability of the recipient to generate apoptotic MSC appears to be a requirement for the therapeutic efficacy and lends itself to be used as a potential biomarker to stratify patients for MSC infusions.

MSC recognition by cytotoxic cells is not antigen-specific as neither requires HLA engagement nor results from an alloreactive rejection, thus supporting the current practice of using third-party MSC. MSC must be in physical contact with the activated cytotoxic cells to undergo apoptosis, although immunological synapse is not required.

The data reported herein suggests an approach to MSC therapeutics that highlights the key role of MSC recipient to orchestrate and determine MSC effector functions. Not only are cytotoxic cells in the recipient required to initiate apoptosis in infused MSC, but also phagocytes which, by engulfing apoptotic MSC and producing IDO, ultimately deliver MSC immunosuppressive activity. Similar mechanisms have been described to explain how apoptotic cells of different lineages, generated *in vitro,* induce immune modulation in GvHD and macrophage IDO production in other systemic autoimmune diseases. This is also consistent with the described ability of MSC to stimulate recipient immune tolerance networks, like regulatory T cells and macrophages.

The depletion of recipient macrophages or the inhibition of IDO activity impairs also the therapeutic activity of live MSC, thereby linking *in vivo* MSC apoptosis with immunosuppression. It is unlikely that any particular phagocyte population (macrophages or dendritic cells) is selectively involved in engulfing apoMSC because they similarly display such an activity *in vivo.*

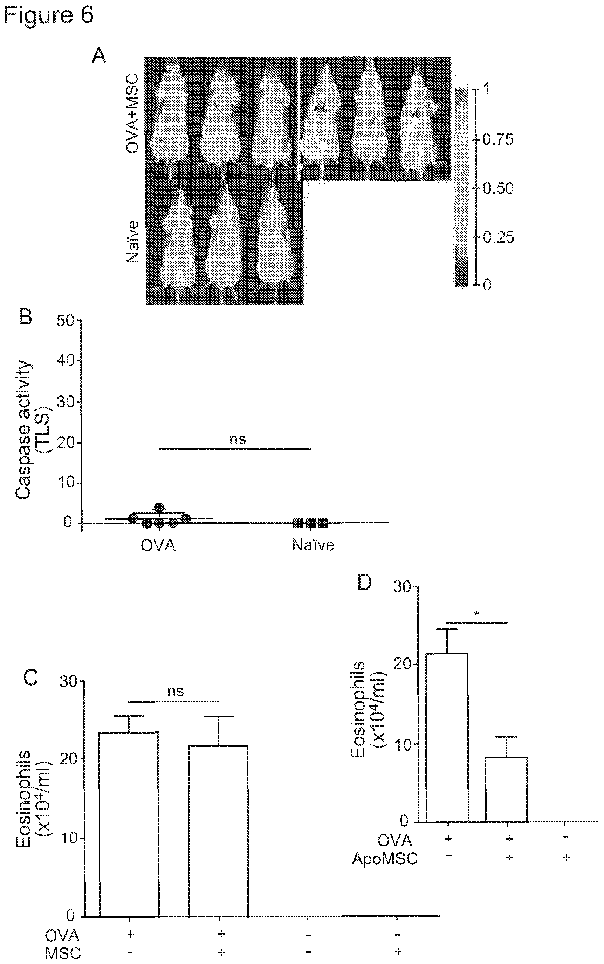

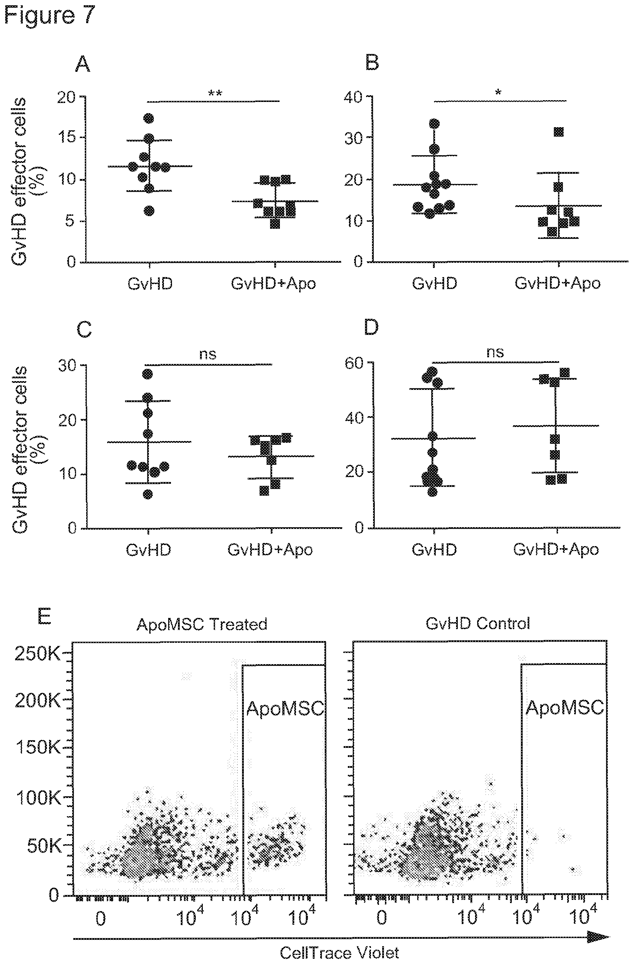

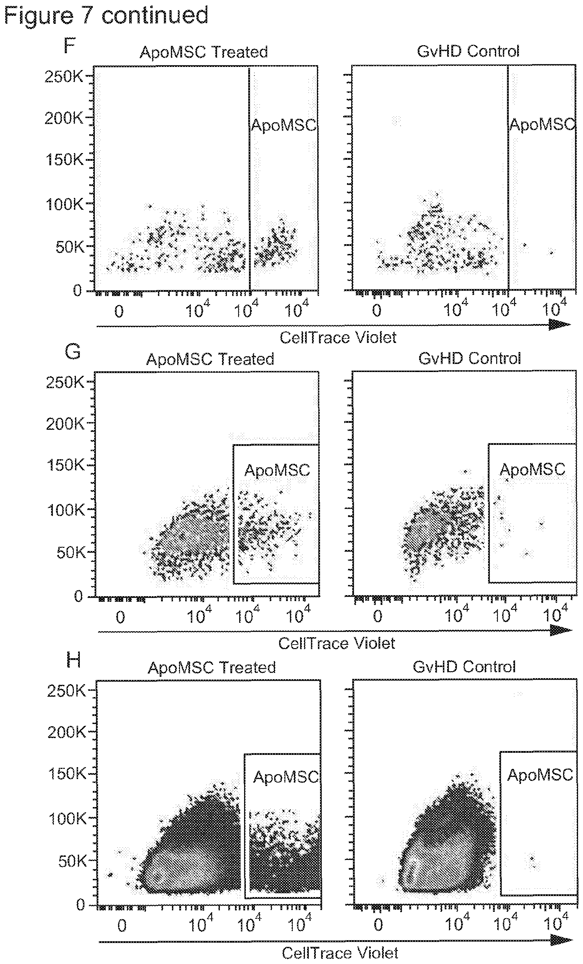

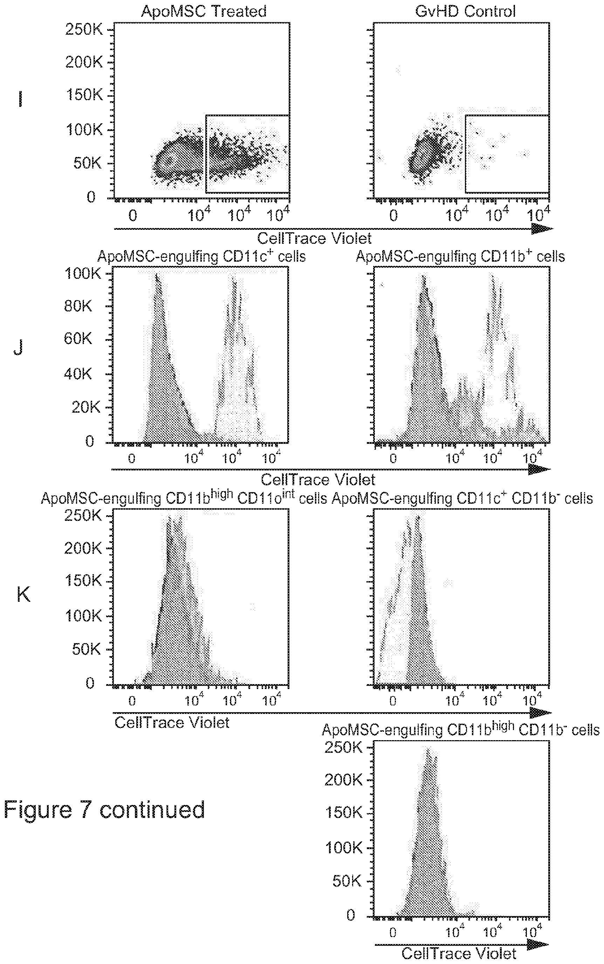

One of the impacts of the present invention is that, although MSC remain the necessary starting point for therapeutic immunosuppression, patient-derived cells play a crucial role in delivering such an immunosuppression. Therefore, the efforts aimed at identifying the most clinically effective MSC subpopulation as well as the potency assays to validate such a selection may prove futile. A further proof supporting this concept is that the administration of *ex-vivo* generated apoMSC can circumvent the requirement for cytotoxic cells in a Th2 inflammatory model (Fig. 6D) and that apoMSC can be effective at suppressing the expansion/infiltration of the GvHD effector cells. Interestingly, apoMSC were mostly effective in the GvHD model only when administered i.p. (Fig. 7, A, B, C, and D). Despite being phagocytosed, apoMSC injected i.v. did not induce IDO production (Fig. 7, J and K), thus suggesting that the site at which MSC apoptosis occurs may influence the immunosuppressive function, perhaps by engaging with a subpopulation of phagocytes.

The present invention represents a paradigm shift in MSC therapeutics whereby their apoptotic demise is a key step in the effector mechanism of immunosuppression exerted by MSC. A further impact of the present invention is that the principle underpinning this mechanism can be used to predict clinical responses to MSC and therefore stratify GvHD patients for MSC treatment.

Patient stratification as described herein, can assist in ensuring that patients receive the most effective treatment available, which may be MSC in some cases, rather than ApoMSC.

The present invention indicates that the next generation of clinical trials should move from choosing the best MSC population to choosing the patients most likely to respond. Furthermore, the finding that apoMSC may be effective in patients refractory to MSC, paves the way to new avenues in the manufacturing of MSC.

### DETAILED DESCRIPTION

### Items

The invention will now be described with reference to the following Items in which:
1. A method for identifying a patient likely to respond to immunosuppressive treatment using live mesenchymal stromal cells (MSCs), said method comprising determining whether a sample from said patient is able to induce at least some apoptosis to occur in live MSCs *in vitro,* and where the ability of the sample to induce said apoptosis is indicative of responsiveness of said patient to said immunosuppressive treatment.
2. A method according to item 1 wherein the ability of the sample to cause apoptosis in MSCs is determined by incubating said sample with live MSCs and thereafter, detecting the presence of apoptotic MSCs in the incubate.
3. A method according to any one of the preceding items wherein a finding that at least 11% of MSCs added to the sample are apopotic after incubation is indicative of responsiveness of said patient to said immunosuppressive treatment.
4. A method according to item 1 wherein the ability of the sample to cause apoptosis in MSCs is determined by incubating said sample with live MSCs, detecting the level of prostaglandin E2 (PGE2) in the incubate or a supernatant obtained therefrom, and relating the level to the responsiveness of said patient to said immunosuppressive treatment.
5. A method according to item 5 wherein a level of PGE2 in excess of 4000pg/ml is indicative of responsiveness of said patient to said immunosuppressive treatment.
6. A method according to item 4 or item 5 wherein the level of PGE2 is determined using a quantitative ELISA.
7. A method according to any one of the preceding items, wherein a patient determined to be responsive to immunosuppressive treatment using live MSCs is prescribed for treatment with MSCs, and wherein a patient determined to be non-responsive to said immunosuppressive treatment is prescribed treatment with apoptotic MSCs.
8. A method of using live mesenchymal stromal cells (MSCs)in vitro, the method comprising contacting the live MSCs in vitro with a biological sample from a patient, and detecting either the presence of apoptotic MSCs among the live MSCs in vitro or elevated levels of prostaglandin E2 (PGE2) in the sample, wherein the presence of apoptotic MSCs among the live MSCs or elevated levels of PGE2 in vitro is indicative of responsiveness of the patient to an immunosuppressive treatment using live MSCs.
9. A method according to item 8, further comprising contacting a plurality of samples of live MSCs *in vitro,* with a biological sample from a different patient in a plurality of patients, and detecting the presence of apoptotic MSCs in each sample of live MSCs *in vitro.*
10. A method according to item 8 or item 9 wherein the or each contacting step comprises incubating the biological sample with live MSCs, and wherein the or each detecting step comprises detecting the presence and/or determining the potency of apoptotic MSCs in the incubate.
11. A method according to any one of items 8 to 10 wherein a finding that at least 11% of the live MSCs in vitro are apopotic after contact with the sample, or that the level of prostaglandin E2 in a supernatant is in excess of 4000pg/ml is indicative of responsiveness of the patient to the immunosuppressive treatment.
12. A method according to any one of items 8 to 11, wherein if the presence of apoptotic MSCs among the live MSCs and/or elevated levels of PGE2 *in vitro* is detected, the patient is treated with live MSCs, and wherein if the presence of apoptotic MSCs among the live MSCs *in vitro* is not detected, the patient is treated with apoptotic MSCs.
13. A method according to any one of the preceding items wherein the sample is a blood or serum sample.
14. A method according to any one of the preceding items wherein the presence of apoptotic MSCs is determined by confocal microscopy or flow cytometry.
15. A method of stratifying patients for treatment with MSC immunosuppression therapy, said method comprising carrying out a method according to any one of items 1 to 14, identifying patients likely to respond to immunosuppressive treatment using live mesenchymal stromal cells (MSCs) for treatment with live MSCs, and identifying those who are not likely to respond to said immunosuppressive treatment for treatment with apoptotic MSCs.
16. A method for identifying a subject's fitness to recover from tissue injury, said method comprising determining whether a sample from said subject is able to induce at least some apoptosis to occur in live MSCs *in vitro,* and/or detecting elevated levels of PGE2, and wherein the ability of the sample to induce said apoptosis, or the presence of elevated levels of PGE2, is indicative of said patient's capacity to recover.
17. A method for producing an immunosuppressive effect in a patient in need thereof, said method comprising determining whether said patient is likely to respond to immunosuppressive treatment using live mesenchymal stromal cells (MSCs), by detecting the presence of at least some apoptosis in live MSCs *in vitro,* in contact with a biological sample from the patient, administering to the patient an effective amount of live MSCs when the presence of apoptosis in the live MSCs *in vitro* is detected, and administering to the patient an effective amount of apoptotic MSCs when the presence of apoptosis in the live MSCs *in vitro* is not detected.
18. A method according to item 17 wherein the patient is suffering from an immune-mediated disease or condition or requires regenerative medicine to stimulate tissue repair.
19. A method according to item 17 or item 18 wherein the disease or condition is allo-immune or autoimmune disease, or is for the prevention or treatment of rejection of a transplanted organ.
20. A method according to item 19 wherein the disease is Graft-vs-Host-Disease, multiple sclerosis, inflammatory bowel diseases such as Crohn's disease or ulcerative colitis, arthritis such as rheumatoid arthritis, ankylosing spondylitis or psoriatic arthritis, and acute respiratory distress syndrome.
21. A method according to any one of item 15 to 20 wherein efficacy of the treatment in the patient is monitored by detecting the presence of phagocytes or IDO in a sample taken from the patient after administration of MSC.
22. A kit comprising components required to carry out a method according to any one of item 1 to 12.
23. Apoptotic MSCs for use in the treatment of immune-mediated disease or conditions or in regenerative medicine to stimulate tissue repair.
24. Apoptotic MSCs for use in a method according to any one of item 7, 12, 15, 17-20.
25. Apoptotic MSCs according to item 23 or 24 wherein the disease is allo-immune or autoimmune disease, or is for the prevention or treatment of rejection of a transplanted organ.
26. Apoptotic MSCs according to item 25 wherein the disease is Graft-vs-Host-Disease, multiple sclerosis, inflammatory bowel diseases such as Crohn's disease or ulcerative colitis, arthritis such as rheumatoid arthritis, ankylosing spondylitis or psoriatic arthritis, and acute respiratory distress syndrome.
27. A method for producing apoptotic MSCs for use in immunosuppression therapy, said method comprising incubating live MSCs with a pharmaceutically acceptable apoptosis inducing agent for a period sufficient to form a cell culture in which at least 30% of the MSCs are apoptotic within a period of less than 24 hours.
28. A method according to item 27 wherein pharmaceutically acceptable apoptosis inducing agent is a biological agent.
29. A method according to item 28 wherein said biological agent is a serine protease and/or an anti-FAS antibody.
30. A method according to item 29 wherein said serine protease is human Granzyme B (GrB).
31. A method according to any one of item 27 to 30 wherein the pharmaceutically acceptable apoptosis inducing agent comprises a combination of GrB and anti-Fas antibody.
32. Apoptotic MSCs obtainable using a method according to any one of item 27 to 31.
33. Apoptotic MSCs according to item 32 for use in immunosuppression therapy.
34. Apoptotic MScs according to item 32 or 33 comprising a suicide mechanism, optionally in the form of a suicide gene, particularly a caspase-inducible gene, such as caspase 9 or caspase 3.
35. A pharmaceutical composition comprising apoptotic MSCs according to any one of item 31 to 33.
36. A composition according to item 35 which further comprises live MSCs.
37. A method for producing an immunosuppressive effect in a patient in need thereof, administering to said patient an effective amount of apoptotic MSCs.
38. A method according to item 37 wherein the patient is suffering from an immune-mediated disease or condition or requires regenerative medicine to stimulate tissue repair.
39. A method according to item 37 or item 38 wherein the disease or condition is allo-immune or autoimmune disease, or is for the prevention or treatment of rejection of a transplanted organ.
40. A method according to item 39 wherein the disease is Graft-vs-Host-Disease, multiple sclerosis, inflammatory bowel diseases such as Crohn's disease or ulcerative colitis, arthritis such as rheumatoid arthritis, ankylosing spondylitis or psoriatic arthritis, and acute respiratory distress syndrome.
41. A method according to any one of item 37 to 40 wherein efficacy of the treatment in the patient is monitored by detecting the presence of phagocytes or IDO in a sample taken from the patient after administration of MSC.
42. Use of prostaglandin E2 (PGE2) and/or use of apoptotic MSCs as biomarkers for determining fitness to recover in a patient and/or responsiveness to immunosuppressive treatment.
43. Use according to item 42, comprising detection of the presence and amount of apoptotic MSCs and/or detecting elevated levels of PGE2 in a sample from said patient.

### Figures

The invention will now be particularly described by way of example with reference to the accompanying drawings which are summarised below.
**Figure 1****. MSC undergo *in vivo* apoptosis after infusion without affecting delivery of immunosuppression.**
   **A:** luc-MSC were injected i.v. into naïve, BM and GvHD mice 3 days after transplantation. All animals were then injected i.p. with DEVD-aminoluciferin and imaged 1 hour later. *N*: 6 mice per group, grouped from 3 independent experiments. White lines separate multiple photographs assembled in the final image. **B:** TLS was measured from the images of mice in Fig.1A and shown as mean±SD. **C, D:** Infiltration of GvHD effector cells (CD8+Vβ8.3+) in the spleen (**C**)and lungs (**D**) of GvHD mice (black circles) and GvHD mice treated with MSC (black squares), 4 days after MSC injection. *N*: 15 (GvHD) and 13 (GvHD+MSC) mice, grouped from 4 independent experiments; mean±SD are shown. Statistics in B: one-way ANOVA, with Tukey's Multiple Comparison Test. **: p<.01, ***: p<.001, ns: not significant. In C and D: unpaired t-test. **: p<.01.

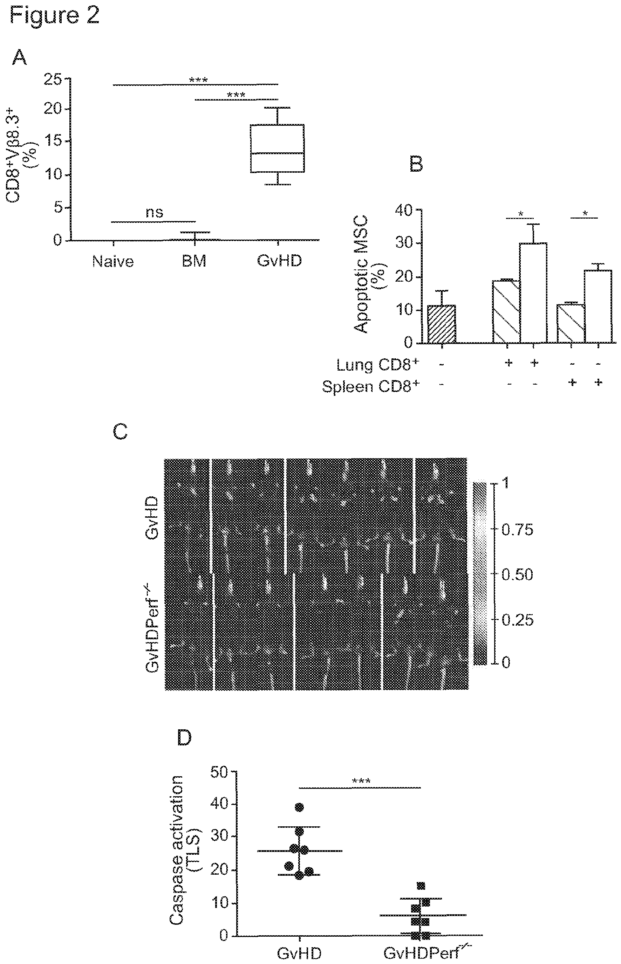

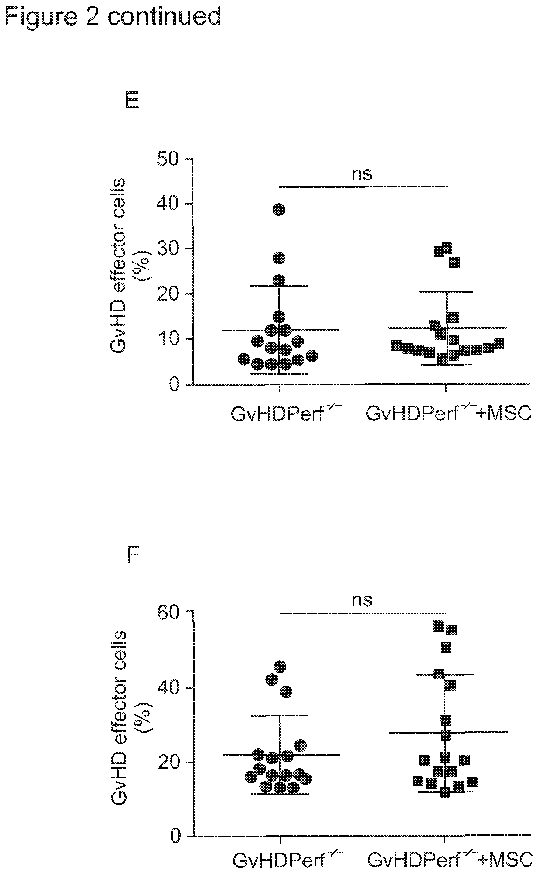

**Figure 2****. MSC apoptosis is indispensable for immunosuppression and requires functionally activated cytotoxic cells in the recipient.**
   **A:** The percentage of CD8+Vβ8.3+ cells in lung cell suspensions from naïve C57BL/6 male, BM or GvHD mice was analyzed in the lymphocyte population; mean±SD are shown. *N*: 12 (GvHD), 3 (BM) and (3(naïve) mice, grouped from 3 independent experiments. **B:** CD8+ cells were sorted from the lungs and spleens of naïve female Mh (grey bars) or GvHD mice (white bars) 7 days after transplant and tested for their ability to induce MSC apoptosis *in vitro.* The results show annexin-V+/7-AAD- MSC (mean±SD) in 3 independent experiments (*N*=10 per group), black bar represents the level of apoptosis in MSC cultured alone used as control (*N*: 3) **C:** luc-MSC were infused in three independent experiments in GvHD (*N*=7) and GvHDPerf-/- (*N*=7) mice 3 days after transplantation. 1 hour later mice were injected with DEVDaminoluciferin and imaged. White lines separate multiple photographs assembled in the final image. **D:** TLS was obtained from Fig. 2C and expressed as mean±SD. **E, F:** infiltration of effector GvHD cells (CD8+Vβ8.3+) in the spleen (**E**) and lungs (**F**) of untreated GvHDPerf-/- (*N*=16) and GvHDPerf-/-(*N*=17) mice treated with MSC (mean±SD of 4 independent experiments). Statistics in A and B: oneway ANOVA, with Tukey's Multiple Comparison Test. *: p<.05; ***: p<.001. In D, E and F: unpaired t-test. ***: p<.001. ns: not significant.
**Figure 3****. Cytotoxic activity against MSC predicts clinical responses to MSC in GvHD patients.**
   **A, B:** PBMC obtained from healthy controls (HC) or patients with GvHD receiving MSC in the following 24 hours were incubated in 24-well plates with MSC at a 20/1 PBMC/MSC ratio for 4 hours. The level of apoptosis was measured in MSC assessing the level of annexin-V/7-AAD by flow-cytometry. (**A**) Representative plots for HC, clinical responders (R) and non-responders (NR). (**B**) The level of apoptosis was compared among HC (circles, *N*=5), R (triangles; *N*=5) and NR (squares; *N*=12). Statistics: one-way ANOVA and Tukey's Multiple Comparison test. ***: p<.0001. ns: not significant.

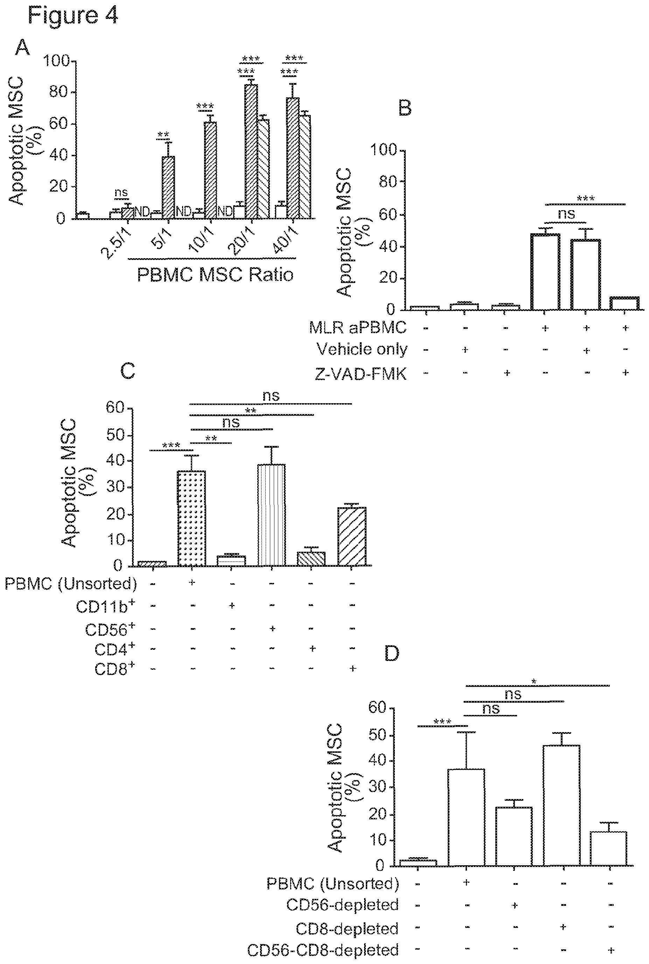

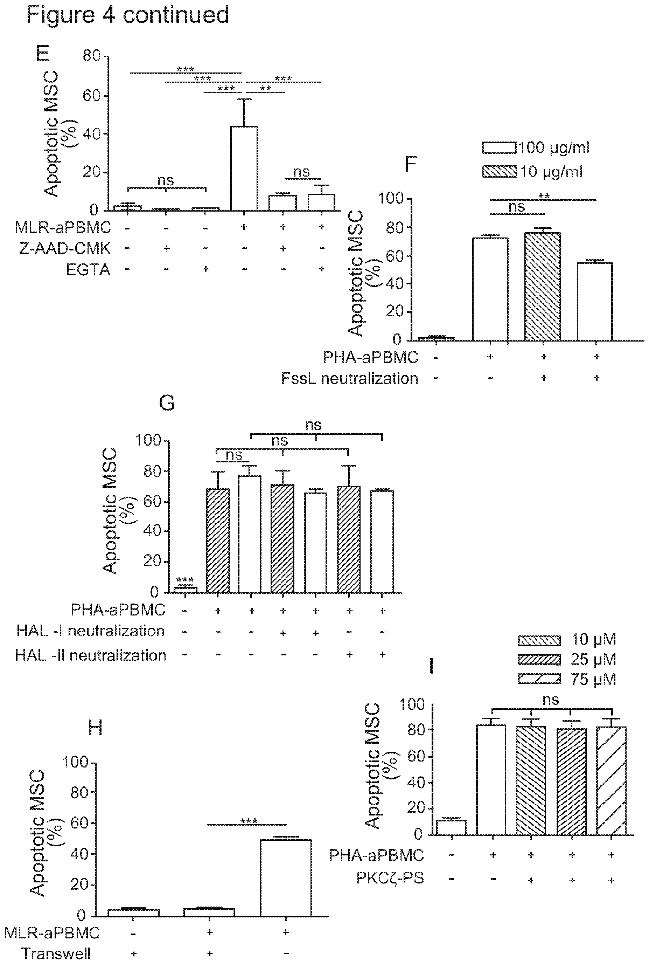

**Figure 4****. MSC apoptosis is mediated by activated CD8+ and CD56+ cytotoxic cells and is the result of a bystander effect.**
   **A:** PBMC from healthy donors were activated using phytohemagglutinin (PHA) (PHA-aPBMC) or MLR (MLR-aPBMC). Resting (light grey bars), PHAaPBMC (black bars) or MLR-aPBMC (dark grey bars) were incubated with MSC at the indicated ratios for 4 hours. ND: Not done. **B, E, F, I:** MLR-aPBMC or PHA-aPBMC were cultivated with MSC in the presence or absence of the pan-caspase inhibitor Z-VAD-FMK (10 µM) (**B**), GrB inhibitor Z-AAD-CMK (300 µM), perforin inhibitor ethylene glycol-bis(2-aminoethylether)-N,N,N',N'- tetraacetic acid (EGTA) (4 mM) (**E**), neutralizing concentrations of FAS-L mAb anti-CD178 (**F**), or escalating doses (10 to 75 µM) of PKCζ-PS (**I**). **H:** MLR-aPBMC were cultivated with MSC in direct contact or separated by a transwell®. **C, D:** MLR-aPBMC were used unfractionated, positively selected for CD11b+, CD4+, CD8+ or CD56+ cells (**C**) or depleted of CD56+, CD8+ or both (**D**). **G:** apoptosis in MSC after culture with autologous (black bars) or allogeneic (grey bars) PHA-aPBMC in the presence or absence of neutralizing doses of anti-HLA-A-B-C or anti-HLA-DR antibodies. In **B-I:** the PBMC/MSC ratio was 20/1. Results represent the mean±SD of 3 or 6 (H) independent experiments. Statistics: one-way ANOVA, with Tukey's Multiple Comparison Test. *: p<0.5. **: p<.01. ***: p<.001. ns: not significant.

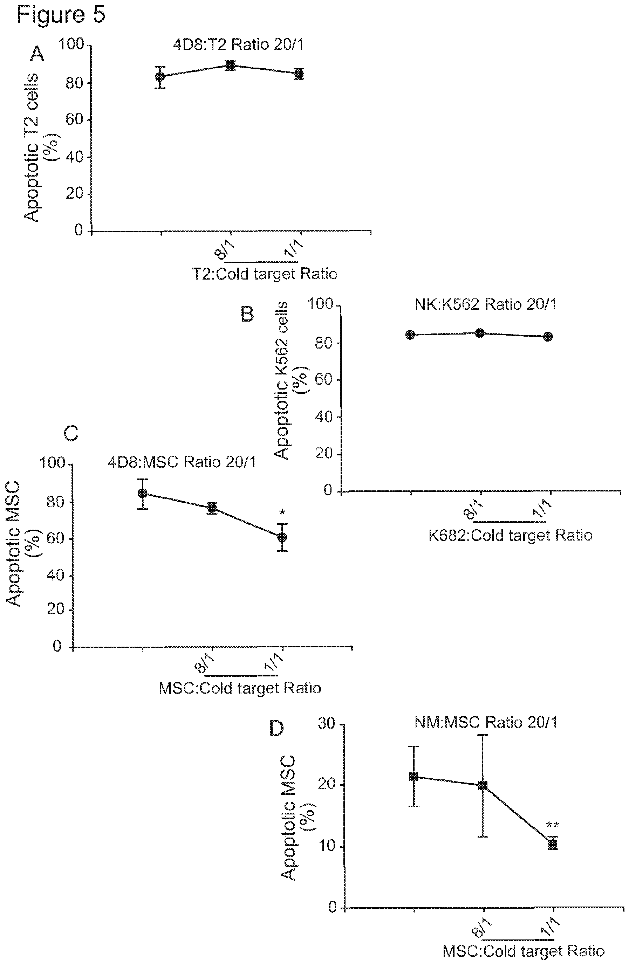

**Figure 5****. MSC apoptosis does not interfere with the antigen-specific cytotoxic cell recognition of the cognate target.**
   **A:** apoptosis in T2-cell after culture with 4D8 cells at a 20/1 4D8:T2 ratio. Where indicated increasing concentrations of MSC (used as *cold* target) were added. Apoptotic T2 cells were identified as annexin-V+/7-AAD+ cells. **B:** apoptosis in K562 cultured with NK cells (20/1 NK:K562 ratio). Where indicated increasing concentrations of MSC (used a *cold* target) were added. **C:** apoptosis in MSC cultured with 4D8 cells (20/1 4D8:MSC ratio). Where indicated increasing concentrations of T2 cells (used as *cold* target) were added. **D:** apoptosis in MSC cultured with NK cells at a 20/1 NK:MSC ratio. Where indicated, increasing dilutions of K562 (used as *cold* target) were added. In all experiments the level of MSC, T2 or K562 cell apoptosis was assessed after 4 hours of co-culture by flow cytometry. Results represent the mean±SD of 3 independent experiments. Statistics in **A, B, C** and **D:** unpaired T-test. *: p<.05. ns: not significant.
**Figure 6****. Apoptotic MSC exert *in vivo* immunosuppressive in a Th2-type inflammation model in the absence of cytotoxic cells.**
   **A:** luc-MSC were injected into naïve (*N*=3) and OVA+MSC (*N*=6) mice one hour after the last challenge. One hour later, mice received DEVDaminoluciferin and were imaged in 3 independent experiments. White lines separate multiple photographs assembled in the final image. **B:** TLS was measured from Fig. 6A (mean±SD). **C:** Eighteen hours after MSC infusion, eosinophil infiltration was assessed in the BAL of naïve (*N*=3), naïve infused with MSC (*N*=3), OVA (*N*=6) and OVA+MSC (*N*=6) mice in two independent experiments and mean±SD are shown. **D:** eosinophil infiltration (mean±SD) in BAL of OVAsensitized mice treated with ApoMSC. Groups were: OVA without ApoMSC (*N*=6), OVA treated with 1x10⁶ ApoMSC (*N*=7); and naïve mice receiving 1x10⁶ (*N*=2) ApoMSC. Results represent the mean±SD of 3 independent experiments. Statistics in **B:** unpaired t-test. ns: not significant. Statistics in **C and D:** one-way ANOVA and Tukey's Multiple comparison test. *: p<.05. ns: not significant.
**Figure 7****. ApoMSC exert immunosuppressive activity in GvHD and are engulfed by recipient phagocytic cells in which they elicit IDO production.**
   **A-D:** Infiltration of GvHD effector cells was assessed in spleen (A, C) and lungs (B, D) of GvHD mice (black circles) and GvHD mice treated with ApoMSC (black squares). ApoMSC were infused i.p. (GvHD mice *N*=10, GvHD+ApoMSC mice *N*=8) (A, B), or i.v. (GvHD mice *N*=9, GvHD+ApoMSC mice *N*=7) (C, D). Results represent the mean±SD of 3 independent experiments. Statistics: unpaired t-test. *: p<.05; **: p<.01. ns: not significant. **E-K:** MSC were labelled using CellTrace™ Violet and subjected to apoptosis induction using GrB/FAS-L (5 µg/ml and 10 µg/ml, respectively). ApoMSC were injected i.p. (E, F and J) or i.v. (G, H, I and K) into GvHD mice 3 days after the transplant. After 2 hours, animals were sacrificed and mesenteric lymph nodes (E, F and J) or lungs (G, H, I and K) were harvested. Cells engulfing ApoMSC were identified as Violet+ cells within the CD11b+(E), CD11c+ (F), CD11bhighCD11cint (G), CD11c+CD11b- (H) and CD11bhighCD11c- (I)subpopulations. The corresponding subpopulations were gated in GvHD mice which had not received violet-labelled ApoMSC. **J** and **K:** IDO expression was assessed in CD11c+ and CD11b+(J) or CD11bhighCD11cint, CD11c+CD11b- and CD11bhighCD11c- (K) cells positive for CellTraceTM Violet (engulfing apoMSC) and compared with the corresponding populations in GvHD mice that had not received ApoMSC. Data are representative of similar results obtained from three mice in 2 independent experiments.

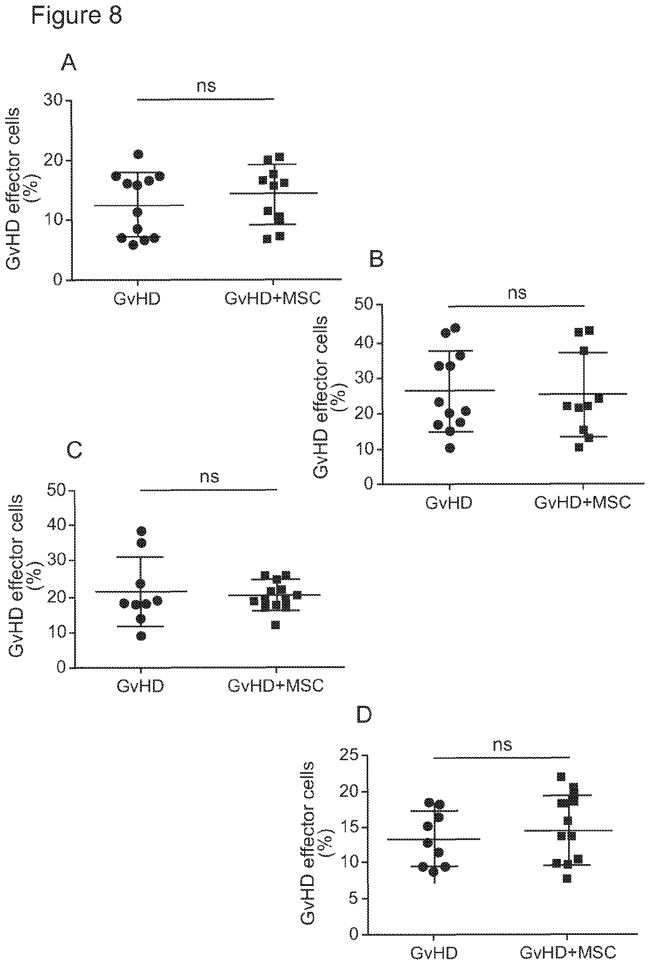

**Figure 8****. Recipient phagocytes and IDO production are required for MSC immunosuppressive activity in GvHD.**
   **A, B:** GvHD mice were treated with liposomal clodronate 10 minutes after the transplant. Where indicated, MSC were infused 3 days later. The infiltration of GvHD effector cells (CD8+Vβ8.3+) in spleen (A) or lungs (B) was quantitated in spleen and lungs after 4 additional days. Mean±SD was obtained grouping three independent experiments with *N*: 12 (GvHD) and 10 (GvHD+MSC) mice per group. **C, D:** GvHD effector cell infiltration was studied in spleen (C) and lungs (D) of GvHD mice treated with the IDO-inhibitor 1-DMT. In the treated mice, MSC were infused 3 days after the transplant (*N*=11). Controls consisted of GvHD mice which did not receive MSC (*N*=9). Results refers to the mean±SD of 3 independent experiments. Statistics: unpaired t-test. *: p<.05; **: p<.01. ns: not significant.

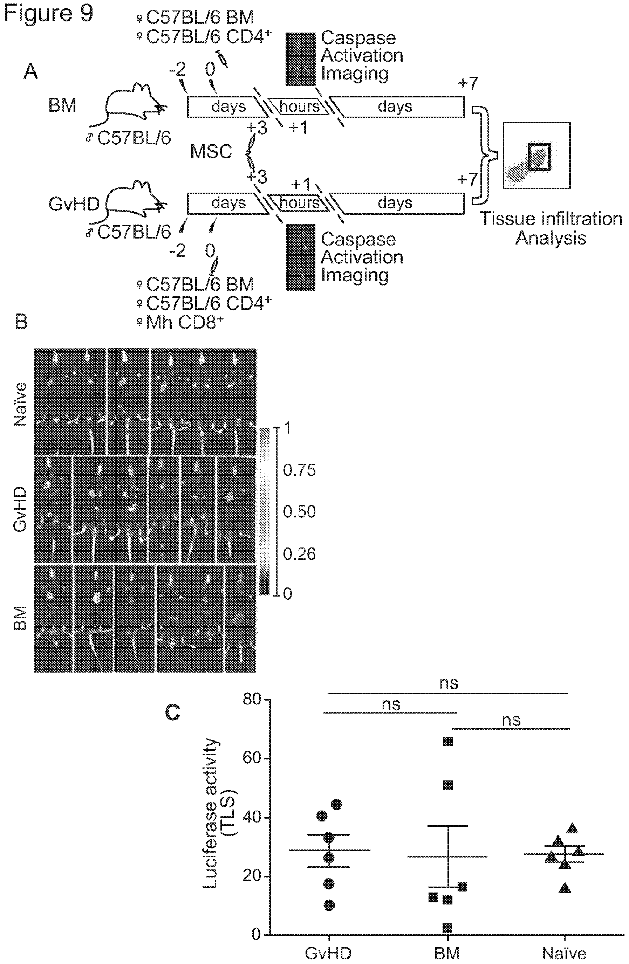

**Figure 9****. MSC can be traced in the lungs of mice after infusion.**
   **A**: lethally irradiated C57BL/6 male mice were transplanted with bone marrow (BM) and CD4+- purified cells from female syngeneic donors with or without CD8+ cells purified from Mh mice (CD8+V*β*8.3+) (GvHD and BM groups, respectively). At day +3 post-transplant,luc-MSC were infused and mice imaged one hour later for the analysis of caspase 3 activation after i.p. injection of DEVD-aminoluciferin. At day +7 posttransplant, mice were sacrificed and the infiltration of GvHD effector cells (CD8+V*β*8.3+) in lungs and spleen was analyzed by flow-cytometry. **B:** in order to confirm the presence of luc-MSC in the lungs of all groups of mice infused with MSC, the same mice imaged in Figure 1A were injected with D-Luciferin. White lines separate multiple photographs assembled in the final image. **C:** TLS was measured from the images of mice in Fig. 9B and shown as mean±SD. Statistics: one-way ANOVA, with Tukey's Multiple Comparison Test. ns: not significant.

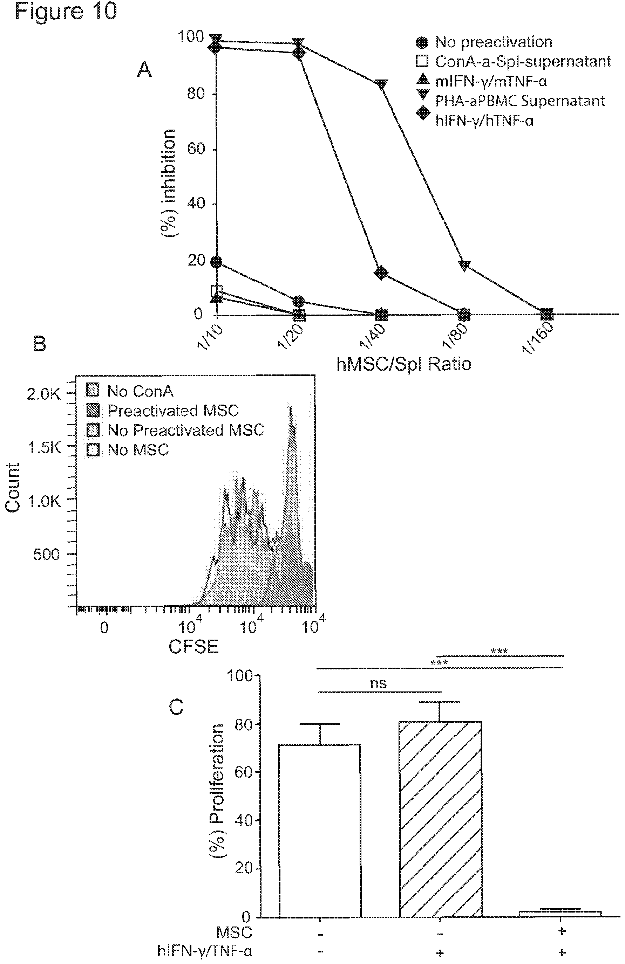

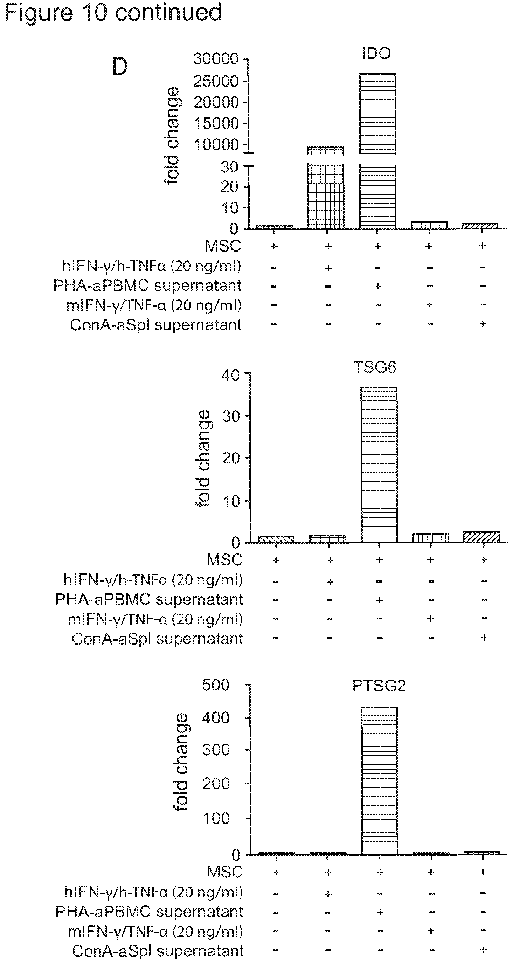

**Figure 10****. Human MSC immunosuppression is not 'licensed' by murine cytokines.**
   **A:** human MSC were plated overnight at serial dilutions alone or in the presence of hIFN-γ/hTNF-α (20 ng/ml each) or mIFN-γ/mTNF-α (20 ng/ml each) or supernatant obtained from PHA-aPBMC for 72 hours or mSpl activated with ConA (ConA-aSpl) for 72 hours, as indicated. MSC were then tested for the ability to inhibit the proliferation of ConA-stimulated mSpl labelled with carboxyfluorescein succinimidyl ester dye. Proliferation was determined after 72 hours by flow-cytometry. The curve was obtained plotting the percentage of inhibition against the corresponding MSC/mSpl ratio. **B, C:** human MSC were plated overnight either untreated or exposed to hIFN-γ/hTNF-α (20 ng/ml each) as indicated and then tested for the ability to suppress mSpl proliferation at 1:10 MSC/mSpl ratio. The histogram plot (**B**) is representative of 3 independent experiments, while bars (**C**) represent the mean±SD of 3 independent experiments. Statistics: one-way ANOVA and Tukey's Multiple Comparison test. ***: p<.001. ns: not significant. **D:** human MSC were incubated alone or in the presence of hIFN-γ/hTNF-α (20 ng/ml each), mIFN-γ/mTNF-α (20 ng/ml each), supernatants obtained from PHA-aPBMC or ConA-aSpl. After 24 hours, *IDO, TSG6* and *PTSG2* expressions were assessed by real time PCR and calculated as relative expression in comparison to untreated MSC. Representative results of three independent experiments are shown.

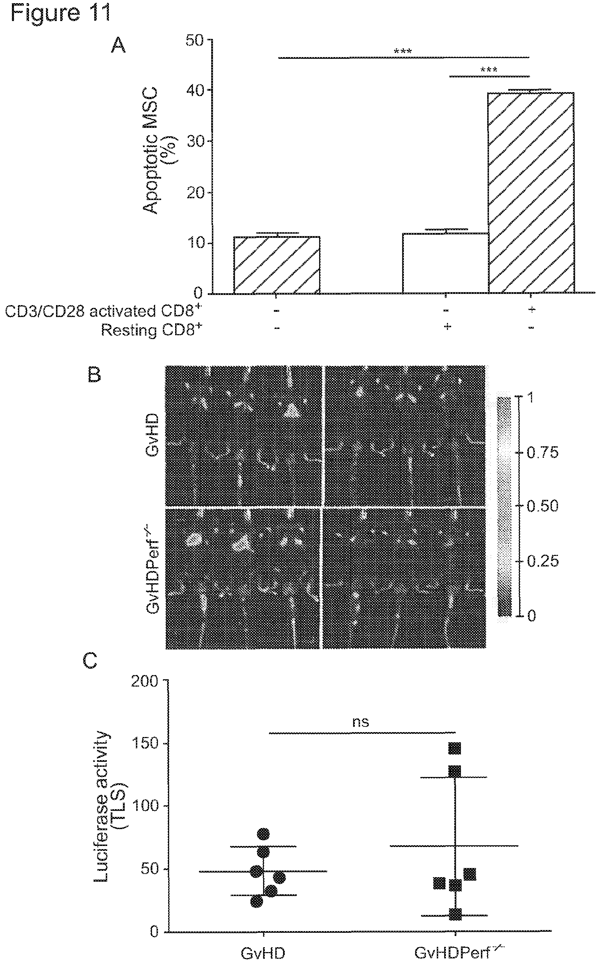

**Figure 11****. MSC apoptosis is activated by cytotoxic cells in a non-antigen specific manner.**
   **A:** CD8+ cells isolated from naïve female Mh mice were stimulated for 3 days with anti-CD3/CD28 beads and cultured with MSC at a 20/1 Mh T-cell:MSC ratio. After 4 hours the level of apoptosis was assessed in MSC by annexin-V/7AAD stainings. Results represent the mean±SD of 3 independent experiments. Statistics: one-way ANOVA, with Tukey's Multiple Comparison Test. ***: p<.001. **B:** in order to confirm the presence of luc-MSC in the lungs of all groups of mice infused with MSC, the same mice imaged in Figure 2C were injected with D-Luciferin. White lines separate multiple photographs assembled in the final image. **C:** TLS was measured from the images of mice in Fig. 11B and shown as mean±SD. Statistics: unpaired t-test. ns: not significant.

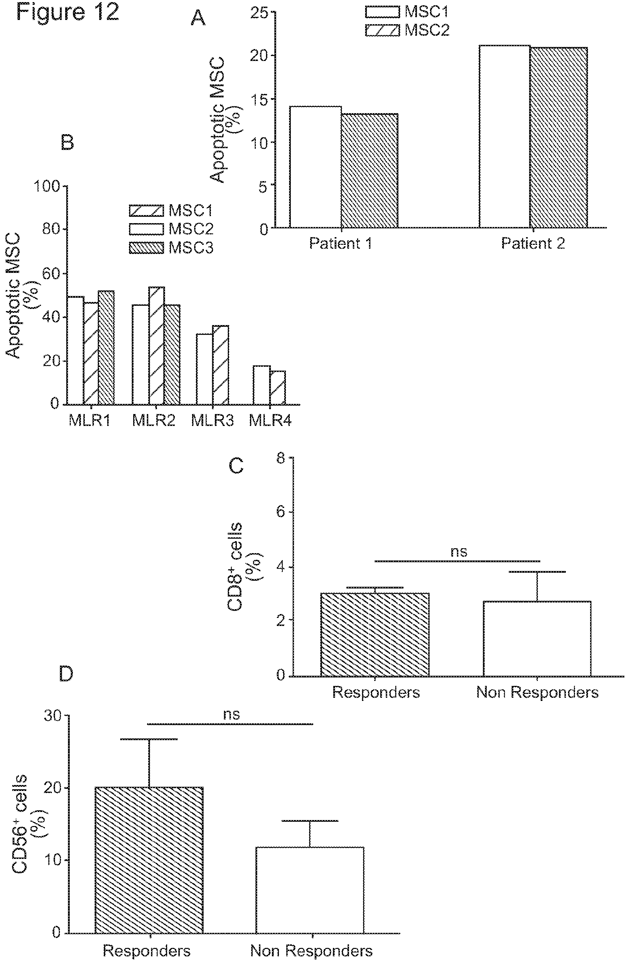

**Figure 12****. Cytotoxicity against MSC varies amongst PBMC donor but is independent on the percentage of CD8+ or CD56+ in GvHD patients.**
   **A:** PBMC obtained from 2 different GvHD patients (Patient 1 and Patient 2) were tested for their cytotoxic activity against MSC from two different donors (MSC1 and MSC2). **B:** apoptosis in MSC obtained from different donors (MSC1, MSC2 and MSC3) after incubation with PBMC from four different MLR responder/stimulator combinations (MLR1, MLR2, MLR3, MLR4). In A and B the level of apoptosis was assessed by flowcytometry after 4 hours of co-culture. **C, D:** PBMC obtained from 11 GvHD patients (R: 3, NR: 8) were analysed for the percentage of CD8+ (**C**) and CD56+ (**D**) cells. Statistics: unpaired t-test. ns: not significant.

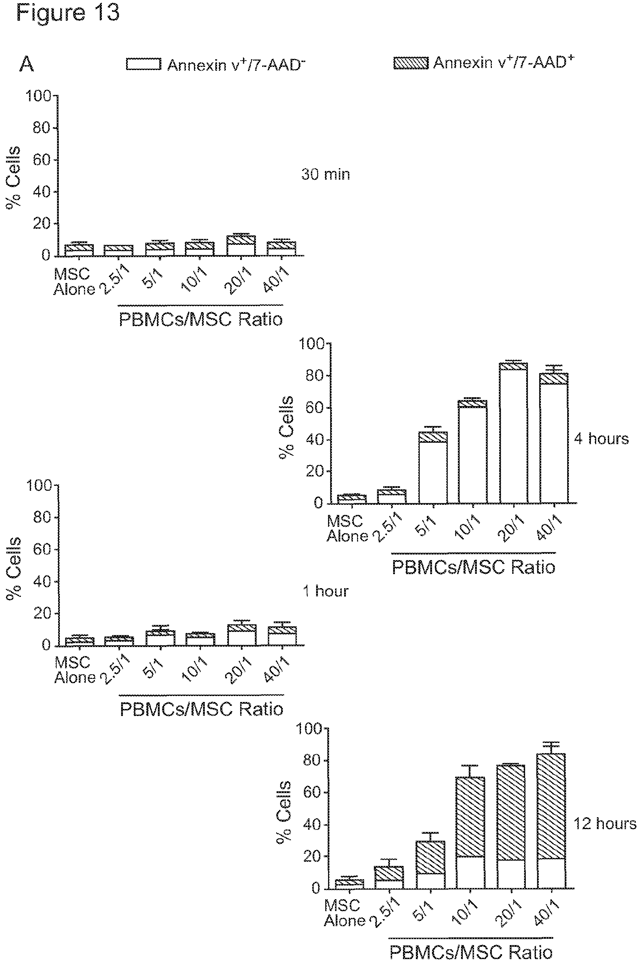

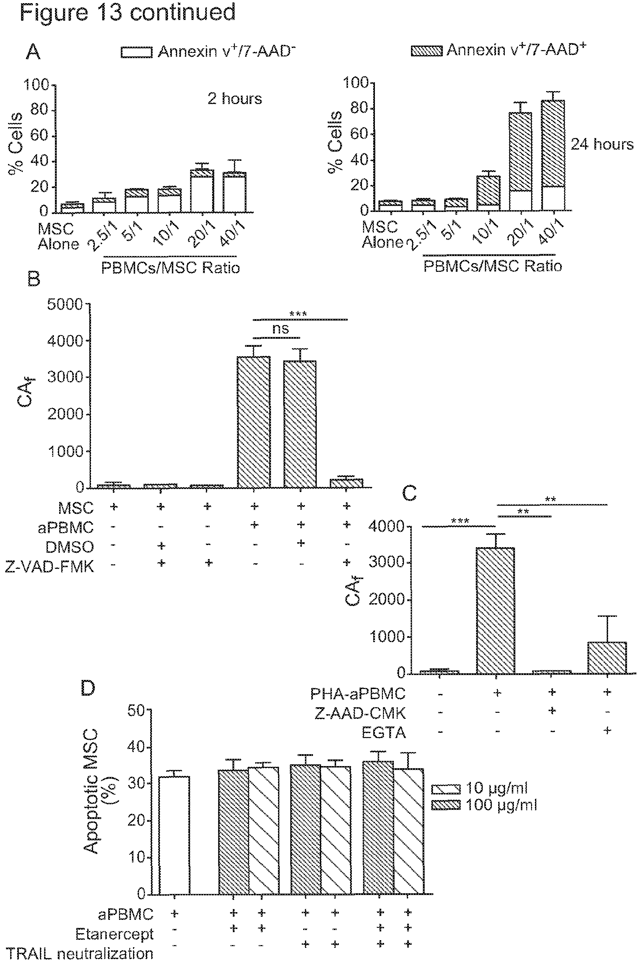

**Figure 13****. MSC killing is mediated by caspase 3 and** effected **by GrB and perforin.**
   **A:** PHA-aPBMC were incubated with MSC at escalating PBMC/MSC ratios. MSC apoptosis was assessed by annexin-V/7-AAD at different time-points by flowcytometry. Results represent the mean±SD of 3 independent experiments. **B, C:** MSC were transfected with the pECFP-DEVDR-Venus vector (FRET-MSC) and FRET between pECFP and Venus-YFP FRET was studied by flow-cytometry and CAf calculated. FRET-MSC were cultured alone, with PHA-aPBMC, or PHAaPBMC in the presence of Z-VAD-FMK (50 µM) (**B**), GrB inhibitor Z-AAD-CMK (300 µM) or the perforin inhibitor EGTA (4 mM) (**C**). Results of 5 (**B**) or 3 (**C**) independent experiments are shown. When PBMC were present, the PBMC:MSC ratio was 40/1. Statistics: oneway ANOVA and Tukey's Multiple Comparison test. **: p>.01. ***: p>.001. ns: not significant. **D**: MLR-aPBMC were cultivated with MSC (20/1 ratio) and apoptosis evaluated by flow-cytometry 4 hours later. Where indicated, the TNF-α inhibitor Etanercept or the mAb anti-TRAIL were used at 10 µg/ml or 100 µg/ml. Results represents the mean±SD of 3 independent experiments.

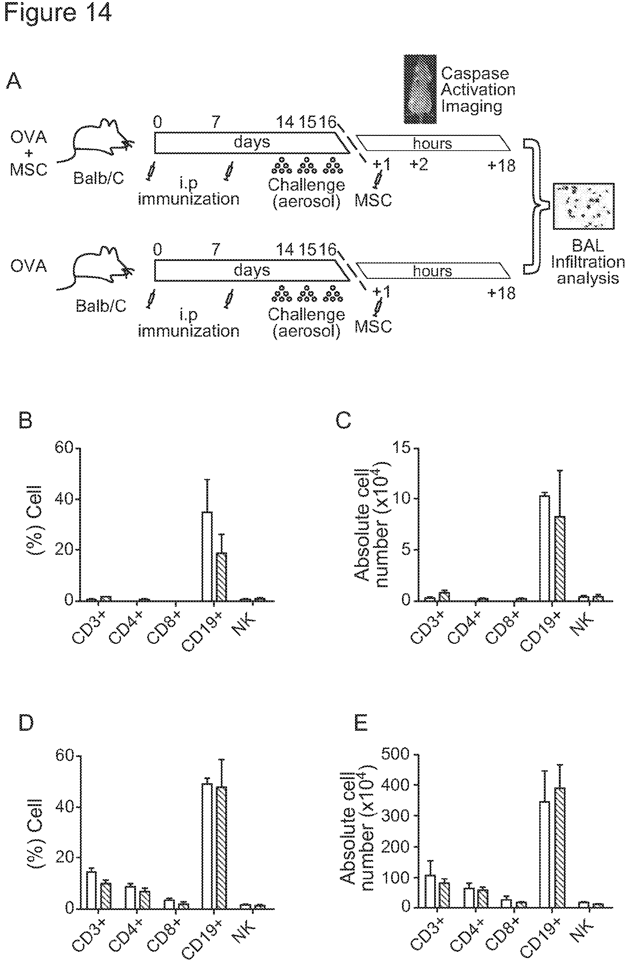

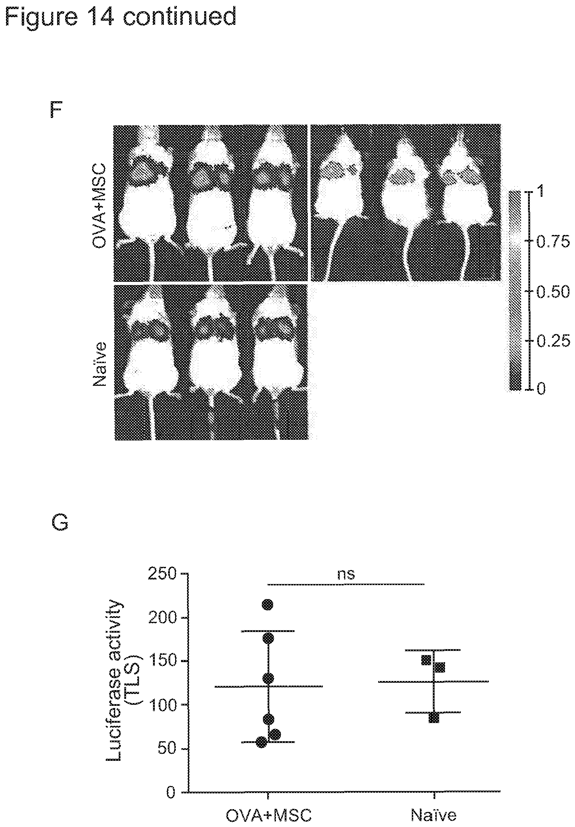

**Figure 14****. Infused MSC can be imaged in the lungs of mice with Th2-type lung inflammation.**
   **A**: Balb/C mice were immunized i.p. with OVA at day 0 and 7 and subsequently challenged with OVA through aerosol at days 14, 15 and 16 (OVA group). Experimental group was treated with MSC one hour after the last challenge (OVA+MSC). When luc-MSC were used, mice were imaged one hour after infusion for the analysis of caspase 3 activation after i.p. injection of DEVD-aminoluciferin. After 18 hours from treatment, eosinophils infiltration in BAL was evaluated. **B-E:** Percentage (**B, D**) and absolute numbers (**C, E**) of different cellular types in the BAL (**B, C**) and lungs (**D, E**) of naïve (with bars) (*N*=3) and OVA-sensitized (black bars) (*N***=**3) mice. Results represent the mean±SD of 3 independent experiments. In OVAsensitized mice, the analysis was performed 1 hour after the last aerosol challenge. **F:** in order to confirm the presence of luc-MSC in the lungs of all groups of mice infused with MSC, the same mice imaged in Figure 6A were injected with D-Luciferin. White lines separate multiple photographs assembled in the final image. **G:** TLS was measured from the images of mice in Fig. 14F and shown as mean±SD. Statistics: unpaired t-test. ns: not significant.

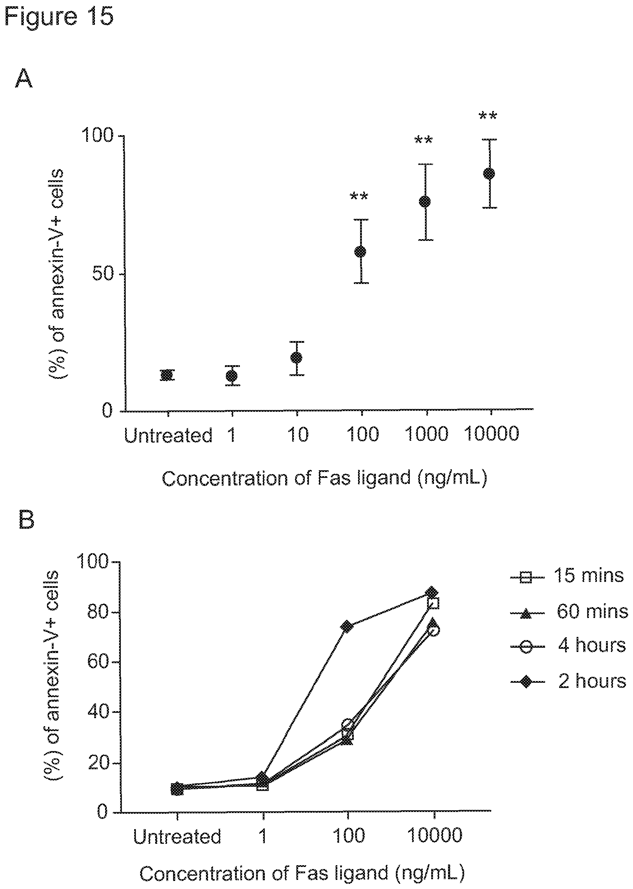

**Figure 15** are a series of graphs showing **(A)** the percentage of ApoMSCs obtained using various concentrations of FasL (anti-Fas antibody), and (**B**) the percentage of ApoMSCs obtained using various concentrations of FasL (anti-Fas antibody) over various time periods.

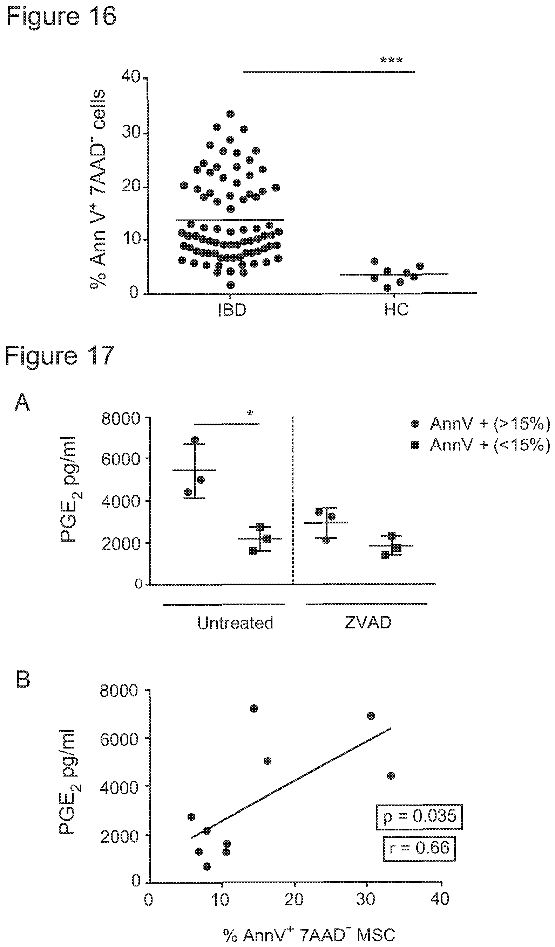

**Figure 16** is a graph showing how PBMC from patients suffering from inflammatory bowel disease (IBD) induce MSC apoptosis ex vivo. PBMC from IBD patients (IBD, n=82) or healthy controls (HC, n=8) were co-cultured with MSC for 4 hours at a 20:1 PBMC:MSC ratio and apoptosis assessed by Annexin-V/7-AAD staining by flow cytometry. Statistics: Unpaired T-test. ***p<0.001.
**Figure 17** is a series of graphs showing how IBD patients induce PGE2 upregulation in MSC and correlate with the proportion of annexinV⁺ MSC. (A) MSC (5 x 10⁴) and patients PBMC (1 x 10⁶) were co-cultured for 24 hours, then the supernatant was collected and PGE2 measured by ELISA. Cells were either left untreated or pre-treated with the pan-caspase inhibitor Z-VAD-FMK (50µM). Statistics: Paired T-test. *p<0.05. **(B)** Correlation between Annexin-V+ MSC and PGE2 levels in the supernatant.

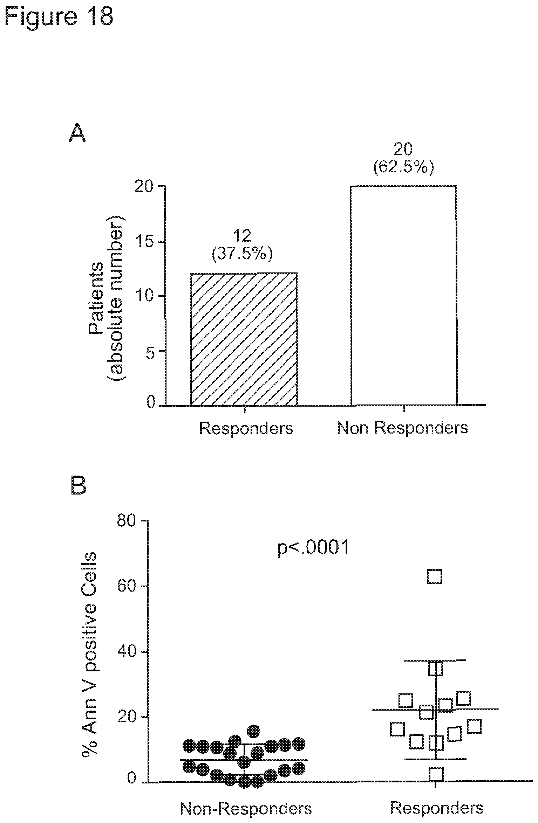

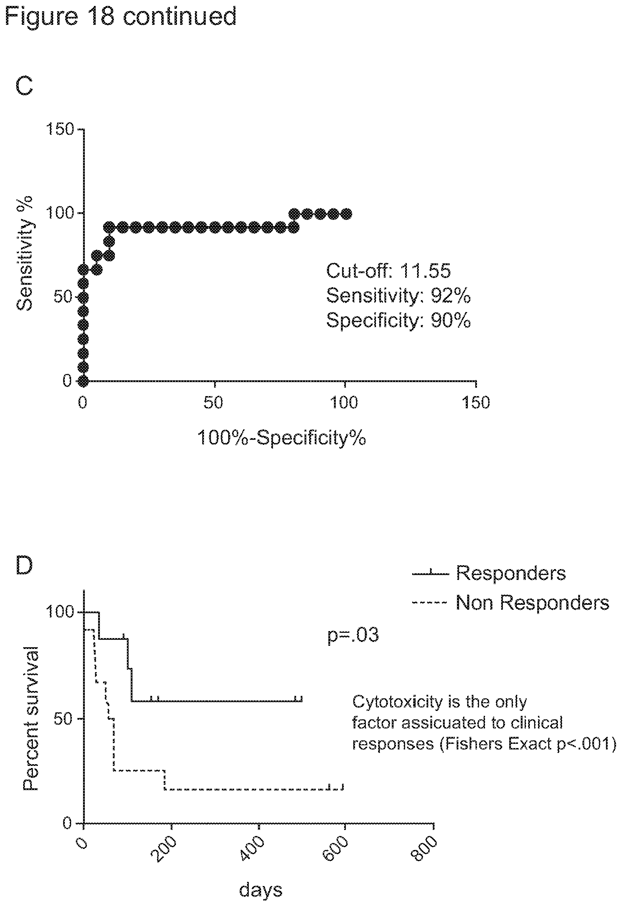

**Figure 18** is a series of graphs showing the ability of patients' PBMC to induce MSC to undergo apoptosis predicts clinical responses to MSC infusions in GvHD. 32 patients affected by steroids-resistant GvHD were treated with intravenous MSC (1-3x10⁶ cells/Kg) (**A**). Before treatment, their peripheral blood mononuclear cells (PBMC) were incubated with MSC for 4 hours and MSC apoptosis was assessed by annexinV staining. The graph reports the proportion of annexinV+ MSC in responders and non-responders (**B**). ROC calculated according to 11.5% MSC killing (annexinV+ MSC) by patients PBMC (C). Overall survival of patients after MSC in responders and non-responders. Amongst the potential factors investigated, cytotoxicity (11.5% threshold) was the only factor influencing clinical response in multivariate analysis.
**Figure 19** shows MSC from different sources exhibiting varying sensitivity to undergo apoptosis. Different batches of MSC isolated from bone marrow (BM) or umbilical cord (UC) were incubated with PHA-activated PBMC from healthy individuals. After 4 hours, MSC were assessed for apoptosis (annexinV+7-AAD-).
**Figure 20** shows PBMC from patients with other inflammatory conditions harbour anti-MSC cytotoxic cells. PBMC were freshly isolated from patients with active inflammatory bowel disease (IBD) or who underwent liver transplantation (Ltx) and co-cultured with bone marrow-derived MSC (20:1 ratio) for 4 hours prior to FACS analysis. Statistic analysis by unpaired t-test (* p<0.05, **p<0.01, ***p<0.001).
**Figure 21** shows that skin fibroblasts are also susceptible to undergo apoptosis when exposed to cytotoxic cells. PBMC isolated from healthy donors were activated for 3 days with PHA and then co-cultured with dermal fibroblasts at different ratios. Apoptotic cells, identified as AnnexinV+ 7AAD- cells, were quantified after 4 hours by FACS analysis.

### Example 1

### MSC undergo apoptosis in recipient GvHD animals.

In order to explain the mechanism by which MSC are rapidly cleared after injection, the applicants tested the hypothesis that MSC undergo apoptosis in a mice model of GvHD.

C57BL/6 (H2b) mice were purchased from Harlan Laboratories (Bicester, UK). Mh (C57Bl/6 background, CD8+Tg, H-2b, CD45.2+, H-2Db-restricted) mice are transgenic for a T-cell receptor specific for the male antigen UTY presented in the context of H-Db, and were bred in-house. All mice were used between 6 and 12 weeks of age.

Acute GvHD was induced as previously described *(*T. Toubai et al. Blood, 119, 3844-3853 (2012*)).* Briefly, after lethal irradiation (11 Gy), recipient C57BL/6 male mice were transplanted with 1x10⁶ purified CD8+ cells transgenic for a T-cell receptor specific for the male HY-antigen Uty (Matahari, Mh) from female Mh mice as GvHD effectors (Figure 9A), 5x10⁶ unfractionated bone marrow (BM) and 2x10⁶ purified polyclonal CD4+ cells from female syngeneic donors (C57BL/6 wild-type donors). The control group received BM and purified CD4+ cells only. CD4+ and CD8+ T cells were obtained by positive selection using magnetic beads (Miltenyi Biotec Ltd, Bisley, UK).

Live MSC (1x10⁶) were injected i.v. at day +3, whilst apoMSC (1x10⁶) were administered i.v. or i.p. at day +1, +3 and +6 from the transplant. Unless otherwise specified, animals were euthanized for analysis at day +7. The infiltration of GvHD effector cells was assessed by flow-cytometry and the percentage was expressed as proportion of cells in the lymphocyte gate, based on the physical characteristics of the cells.

In this model, the expansion of the T cells effecting GvHD (CD8+Vβ8.3+) can be precisely enumerated.

*In vivo* MSC caspase activation was evaluated using MSC that were transfected with the pGL3-control vector for the expression of firefly luciferase (*Luc*+) (luc-MSC). Caspase activation was measured as luciferase activity using DEVD-aminoluciferin. In this system, caspase 3 activation could be quantified on the basis of emitted light since DEVD is cleaved upon activation of caspase 3, leading to release of aminoluciferin which in turn can be metabolized by the firefly luciferase expressed in MSC. Luc-MSC were injected into recipients of BM transplant with CD8+ Mh T cells (GvHD group) and one hour later caspase activity was measured as total luminescence signal (TLS). Control mice consisted of naïve males (naïve group) and a group of mice which were irradiated and received CD4+ and BM cells (BM group) without the transgenic T cells to reproduce the condition of MSC infusion in the absence of activated cytotoxic T cells (Fig. 9A). High caspase activity was observed only in MSC injected into GvHD mice (Fig. 1, A and B). High signal could be detected from the lungs of all animals when the control D-luciferin (firefly luciferase substrate) was used (Fig. 9, B and C), thus confirming that luc-MSC can be tracked in the lungs also when caspase activity could not be detected.

The evidence that MSC undergo apoptosis after infusion prompted the question of whether they are still capable of suppressing antigen-driven T cell expansion. Therefore, their immunosuppressive effect was analysed by enumerating CD8+Vβ8.3+ Mh T cells (GvHD effector cells) in MSC treated or -untreated GvHD mice. MSC produced a significant reduction in GvHD effector cell infiltration in both spleen and lungs (Fig. 1, C and D). These results indicate that, despite the presence of MSC apoptosis after infusion (Fig. 1, A and B), MSC immunosuppression still occurs.

The possibility that the observed immunosuppressive activity could be the consequence of the recipient inflammatory cytokines can be excluded because in our xenogeneic combination murine inflammatory cytokines will not cross-react with the corresponding human receptors and will not activate immunosuppressive molecules in human MSC, whilst retaining the ability to expand murine effector cells mediating GvHD. Accordingly, human MSC were not able to inhibit concanavalin-A (ConA) induced proliferation of murine splenocytes (mSpl) unless pre-activated by human cytokines (Fig. 10, A, B and C). Furthermore, exposure of human MSC to murine inflammatory cytokines did not upregulate *IDO,* TNF-stimulated gene 6 protein (*TSG-6*) or prostaglandin-endoperoxide synthase-2 (*PTSG2*), considered major effectors of human MSC mediated *in vitro* immunosuppression (Fig. 10 D).

### Example 2

### In vivo MSC apoptosis depends on activated recipient GvHD effector cells

Our results show that MSC rapidly undergo apoptosis after infusion, providing the long-sought after explanation for the rapid clearance of transplanted MSC in the recipient. The absence of *in vivo* MSC apoptosis in naïve and BM mice clearly demonstrates that MSC apoptosis is not the result of xenogeneic recognition of human MSC, because it is detected only in GvHD mice. When we enumerated GvHD effector cell infiltrate (CD8+Vβ8.3+) in the lungs of mice, where MSC apoptosis occurs, we found that only the lungs of GvHD but not naïve and BM mice contained a large proportion of CD8+Vβ8.3+ cells (Fig. 2A), thus confirming the correlation between caspase activation in MSC and the presence of GvHD effector cells.

To test the hypothesis that GvHD effector cells were responsible for MSC apoptosis, MSC were cultivated with CD8+ T cells purified from the lungs or spleens of GvHD (*in vivo* activated) or naïve Mh (*in vivo* resting) mice. Activated, but not resting, Mh CD8+ cells induced MSC apoptosis (Fig. 2B). In further support of the lack of antigen-specificity in the induction of the killing activity, high levels of cytotoxicity could be elicited by naïve Mh CD8+ cells stimulated *in vitro* by CD3/CD28 beads (Fig. 11A).

The requirement of cytotoxic cells in the induction of MSC apoptosis and the consequent immunosuppression was evaluated using Mh/Perforin Knock-Out mice (Mh/Perf-/-) as donors of defective cytotoxic GvHD effector cells (GvHDPerf-/- group). C57BL/6-Prf1tmlSdz/J (Perforin-/-) mice were purchased from J Jackson labs, bred with Matahari Rag2-/- mice and the resulting offspring intercrossed for 2 generations to obtain the Mh Rag2-/-.Perf KO F3 mice.

Luc-MSC were infused into GvHDPerf-/- or control GvHD mice which had received Mh CD8+ T cells. Mice were imaged 1 hour later and caspase activation measured as described before. We observed much lower caspase activity in GvHDPerf-/- mice compared to GvHD controls (Fig. 2, C and D). High signal was detected in the lungs of all animals when the control D-luciferin was used (Fig. 11, B and C), thus confirming that luc-MSC were in the lungs also when caspase activity could not be detected. Importantly, the infiltration of GvHD effector cells in the spleen and lungs of mice receiving MSC was not reduced in GvHDPerf-/- receiving MSC (Fig. 2, E and F). We conclude that MSC apoptosis is indispensable for immunosuppression and requires functionally activated cytotoxic cells in the recipient.

### Example 3

### Cytotoxic activity against MSC is a biomarker predictive of clinical response to MSC in GvHD patients

Based on these findings, we inferred that the presence of cytotoxic cells in the recipient could be predictive of MSC therapeutic activity.

16 patients (mean age 40.5 years (range: 10-69), with severe steroid resistant grade 3-4 GvHD were treated with MSC in various hospitals over a period of years. MSC were administered for compassionate use (according to Regulation (EC) No 1394/2007). Patients had received a myeloablative or reduced-intensity conditioning prior to hematopoietic stem cell transplantation. All patients received GvHD prophylaxis with 3 or 4 doses of methotrexate combined with cyclosporine. T-cell depletion with alemtuzumab or ATG was performed in all adult patients transplanted in the UK centers. Of the 16 patients included in the study, 13 developed GVHD following hematopoietic stem cell transplantation, and the remaining 3 after DLI. 12 patients were affected by acute GvHD, 3 by late onset acute GvHD and 1 by chronic GvHD. The diagnosis of GvHD was made on histological criteria and GvHD staged according to standard criteria. Patients were considered to be steroid-refractory if: (a) those with aGVHD failed to respond to high-dose methylprednisolone after 6 days; (b) the one with cGVHD failed to respond to high-dose steroids after 2-4 weeks, with the addition of Mycophenol Mycophenolate Mofetil (MMF) and cyclosporine at 1 and 4 weeks respectively. Clinical responses to MSC were assessed 1 week after MSC infusion and defined as an improvement of at least 50% in at least one organ affected by GvHD. Patient characteristics are summarized in Table 1 hereinafter.

Clinical responses to MSC were defined by an improvement of at least 50% in at least 1 organ affected by GvHD as previously described. Five patients obtained a clinical response.

Peripheral blood mononuclear cells (PBMC) were freshly collected within the 24 hours preceding the MSC infusion and tested directly for their ability to induce MSC apoptosis *ex vivo* in a 4-hour cytotoxic assay (see below). One patient received two doses of MSC and the cytotoxic assay was performed before each dose independently. MSC were sourced from the same donor used for the infusion (*N*=8) or from a different donor (*N*=9). At the time of performing the assay and cytofluorimetric analysis the operator was blind to patients' clinical details. PBMC from healthy donors (*N*=5) were used as controls.

Overall, PBMC from GvHD patients exhibited an average cytotoxic activity against MSC higher than that detected in control PBMC but without reaching a significant difference (mean±SD were: 10.63±8.76% and 3.82±2.50%, respectively; p=.10). However, the level of cytotoxicity between clinical responders and non-responders to MSC was markedly different, with the proportion of apoptotic MSC (annexin-V+/7AAD-) exhibiting a four-fold difference (Fig. 3, A and B). The discrimination threshold of apoptotic MSC between responders and non-responders calculated using the receiver-operating characteristic curve revealed that a 14.85% cut-off was predictive of clinical response with the highest sensitivity and specificity. The level of cytotoxicity did not vary amongst MSC preparations, because when we tested patients' PBMC against the MSC used for the infusion as compared to another preparation obtained from an unrelated donor, no difference in apoptosis induction could be detected (Fig. 12A). To further confirm the irrelevance of the specific MSC preparation, we evaluated the susceptibility of MSC sourced from different unrelated donors to be killed by 4 different mixed lymphocyte reaction (MLR) combinations. The proportion of apoptotic MSC was similar amongst the different MSC preparations when the same MLR was tested. Conversely, the cytotoxic activity against the same MSC varied amongst different MLR (Fig. 12, B).

Finally, we ruled out the possibility that different proportions of CD8+ and CD56+ cells could account for the differing cytotoxic activity because the average frequency in the PBMC of responders and non-responders was similar (Fig. 12, C and D). Therefore, we conclude that the presence of activated cytotoxic cells in the recipient is predictive of MSC therapeutic activity.

### Example 4

### MSC apoptosis induced by cytotoxic cells is the result of a bystander effect

To define the mechanisms that drive apoptosis in MSC, we used *in-vitro*-activated PBMC as effector cells. We found that activated but not resting PBMC induced extensive early apoptosis (annexin-V+/7AAD-) in MSC (Fig. 4A), which peaked at 4 hours and shifted towards late apoptosis (annexin-V+/7AAD+) by 24 hours (Fig. 13, A). In accord with our *in vivo* observations (Fig. 1, A and B), only activated PBMC induced caspase activation in MSC with a peak at 90 minutes, and this was completely abrogated by the pan-caspase inhibitor Z-VAD-FMK (Fig. 4B and Fig. 13B).

In order to identify the cells inducing apoptosis in MSC, we performed selective enrichment and depletion experiments amongst activated PBMC. We found that CD56+ natural killer (NK) and CD8+ populations were the only cells responsible for initiating MSC apoptosis (Fig. 4, C and D). To characterize the mechanisms mediating MSC apoptosis induced by activated cytotoxic cells, we studied potential factors involved in caspase 3 activation. Inhibition of either Granzyme B (GrB) or perforin completely abolished the ability of activated PBMC to kill MSC (Fig. 4E) and activate caspase 3 (Fig. 13C). We also observed reduced PBMC mediated cytotoxicity when CD95 ligand (CD95L, also known as FasL or APO-1L) was neutralized (Fig. 4F), but not when Tumor Necrosis Factor-a (TNF-a) or TNF-related apoptosis-inducing ligand (TRAIL) were inhibited, even in the presence of very high concentrations of their respective inhibitors (Fig. 13D).

We then interrogated the nature of the MSC-cytotoxic cell interaction. We observed that apoptosis was not affected by the presence of anti-HLA class I- or anti-HLA class II neutralizing antibodies. Consistently, the cytotoxic activity of activated PBMC on autologous or allogeneic MSC did not differ (Fig. 4G). However, although PBMC required physical contact with MSC to induce apoptosis (Fig. 4H), blocking immunological synapse formation by inhibiting the polarization of microtubule organizing center (Fig. 4I) had no effect. These results demonstrate that MSC killing by activated cytotoxic cells is a bystander effect that does not involve the immunological synapse.

### Example 5

### MSC apoptosis does not interfere with the recognition of the specific target of cytotoxic cells

Having determined that the MSC apoptosis induced by cytotoxic cells is MHC-independent and not antigen-specific, we asked whether MSC could exert their immunosuppressive effects by competing with and antagonizing antigen-specific recognition. NY-ESO1-specific CD8+ T cell clone (4D8) or IL2-activated polyclonal CD56+ purified NK cells were used as effector cells against NY-ESO-1 peptide pulsed T2 or K562 cells, respectively. Two different sets of experiments were performed. In the first set, 4D8 or NK cells were tested against fixed numbers of putative (*susceptible*) target cells in the presence of escalating numbers of MSC used as a *cold* target. The alternative condition consisted of escalating the numbers of the putative target cells - now used as *cold* targets - in the presence of a fixed number of MSC then considered as the *susceptible* target. MSC did not compete with antigen-specific T cell cytotoxicity, since the killing of peptide-pulsed T2 cells was not affected by the presence of MSC (Fig. 5A). The same results were obtained using NK cells (Fig. 5B). In contrast, the presence of the putative target cells markedly reduced MSC killing in a dose dependent manner in both systems (Fig. 5, C and D). Our data show that MSC killing does not interfere with the primary recognition of the cognate antigen.

### Example 6

### Apoptotic MSC are immunosuppressive in a Th2-type inflammation model

Our data imply that, since MSC killing does not interfere with the primary recognition of the cognate antigen, induction of apoptosis must be prominently involved in the immunosuppressive activity.

Accordingly, in the GvHD model described, MSC apoptosis produced by recipient cytotoxic cells is required for immunosuppression. Therefore, we asked whether this causative relationship remains valid in a different disease model associated with non-cytotoxic Th2-type inflammation. We selected the model of ovalbumin (OVA)-induced allergic airway inflammation summarized in Fig. 14A.

OVA-induced airway inflammation was induced as previously described (Y. Riffo-Vasquez et al., 2012, Am J. Respir. Cell Mol Biol 47, 245-252). Briefly, female Balb/C mice (Harlan Laboratories, Bicester, UK) were injected intra peritoneally with 30 µg of chicken egg albumin (OVA type V) (Sigma-Aldrich Company Ltd, Dorset, UK) on day 0 and 7. Controls received vehicle (aluminum hydroxide) only. On day 14, 15 and 16 animals were challenged with an aerosolized solution of OVA (3%) for 25 minutes. MSC or ApoMSC were injected 1 hour after the last challenge. After additional 18 hours, mice were terminally anaesthetized, a cannula inserted into the exposed trachea and three aliquots of sterile saline were injected into the lungs. The total number of cells in the lavage fluid was counted. For differential cell counts, cytospin preparations were stained with Diff Quick (DADE Behring, Germany) and cells counted using standard morphological criteria.

Although cytotoxic immune cells have been implicated in the induction of this condition,CD8+ and NK1.1+ cells infiltrating bronchoalveolar lavage (BAL) and lung tissues were less than 2% one hour after the last challenge, when MSC were infused (Fig. 14, B, C, D and E). To confirm the absence of MSC killing, mice received luc-MSC to assess caspase activation after infusion and imaged one hour later. No caspase activation was detected in any of the mice (Fig. 6, A and B).

High signal could be detected in all animals receiving control D-luciferin (Fig. 14, F and G). The therapeutic activity, assessed by quantitating the eosinophil infiltration in the BAL showed no difference between MSC-treated and untreated mice (Fig. 6C). Together, these results indicate that also in this model MSC immunosuppression relies on the presence of recipient cytotoxic cells that mediate MSC apoptosis.

Therefore, we decided to test whether *in vitro* generated apoptotic MSC (apoMSC) could bypass the need of cytotoxic cells and ameliorate eosinophil infiltration.

Conditions under which a cell culture containing a significant (>30%) number of apopotic cells was investigated. MSCs were plated at a concentration of 5x10⁵ cells per well in a 96 round-bottom well plate in the presence of synthetic human GrB (5 µg/ml) (Enzo Life Sciences, Exeter, UK) with various concentrations of anti-FAS human (activating, clone CH11) for various times ranging from 15 minutes to 24 hours. After that, the percentage of apoptotic cells were determined by flow cytometry using annexin V staining. The results are summarised in Figure 9.

Following this, ApoMSC were obtained for the test by plating 5x10⁵ cells per well in a 96 round-bottom well plate in the presence of synthetic human GrB (5 µg/ml) (Enzo Life Sciences, Exeter, UK) and anti-FAS human (activating, clone CH11) (10 µg/ml) (Merk Millipore, Watford, UK) for 24 hours in complete RPMI. The concentration of GrB and FasL was chosen to produce at least 80% of MSC apoptosis.

When apoMSC were administered to recipient mice a significant reduction of the eosinophil infiltration in BAL was observed (Fig. 6D).

### Example 7

### Apoptotic MSC infused in GvHD are immunosuppressive and induce IDO production in recipient phagocytes

We subsequently investigated whether apoMSC could be immunosuppressive also in the GvHD model. ApoMSC were administered either intravenously (i.v.). or intraperitoneally (i.p.). and the infiltration of CD8+Vβ8.3+ Mh T cells was assessed and compared to untreated GvHD mice. ApoMSC produced a significant reduction in GvHD effector cell infiltration in both spleen and lungs, but this could only be observed in those mice treated with ApoMSC infused i.p. (Fig. 7, A, B, C and D). It has been reported that the injection of irradiated thymocytes into animals results in their phagocytosis by recipient macrophages and induction of IDO.

We therefore tested whether apoMSC followed the same destiny by eliciting *in vivo* efferocytosis by recipient phagocytes and inducing IDO production. For this purpose, labelled apoMSC were traced in recipient phagocytes after injection.

Specifically, MSC were first labelled using CellTraceTM Violet labelling (ThermoFisher Scientific, Paisley, UK) at a final concentration of 5 µM and then made apoptotic (ApoMSC) as described above, using synthetic human GrB (5 µg/ml) and anti-FAS human (10µg/ml) for 24 hours. 10x10⁶ labelled apoMSC were then injected i.p. or i.v. and mice sacrificed after 2 hours post-injection. Spleen, lungs, peritracheal, paratracheal, pericardial, mesenteric, periportal and celiac lymph nodes were collected and analysed by flow-cytometry. Positivity of CellTraceTM Violet was assessed as measure of ApoMSC engulfment in CD11b+ and CD11c+ gated subpopulations of phagocytic cells. Cells positive for the CellTraceTM Violet were then assessed for their expression of IDO.

Following i.p. administration, apoMSC were largely identified inside CD11b+ (Fig. 7, E) and CD11c+ (Fig. 7, F) phagocytes in the peritoneal draining lymph nodes (Fig. 7, E) but absent when searched for in the lungs and spleen. When the i.v. route was used, amongst the several phagocytic populations investigated, CD11bhighCD11cint, CD11bhighCD11c- and CD11b-CD11c+ were detected as engulfing apoMSC in lungs (Fig. 7, G, H and I, respectively). The analysis of IDO expression in the phagocytes engulfing ApoMSC both in the i.v. and i.p. groups revealed that only the phagocytes in the i.p. group were able to increase IDO expression at a significantly higher level in comparison with their counterparts in untreated GvHD mice (Fig. 7, J and K). These findings strongly suggest that the immunosuppressive effect of apoMSC involve recipient phagocytes and IDO as crucial effector mechanisms.

### Example 8

### Recipient derived IDO-producing phagocytes are indispensable for MSC immunosuppression in GvHD

To directly test the importance of recipient-derived phagocytes and recipient-produced IDO in MSC immunosuppressive activity, we depleted phagocytes and inhibited IDO activity in GvHD mice before MSC treatment and evaluated the effect of live MSC on the expansion of GvHD effectors. To deplete phagocytes, liposome clodronate (1mg) was given to mice 72 hours before MSC injection. The treatment, dramatically impaired the ability of MSC to suppress Mh T cell infiltration (Fig. 8, A and B).

Finally, animals were given the IDO inhibitor 1-methyl-D-tryptophan (1-DMT) (2mg/ml) in the drinking water starting 6 days prior to MSC injection until the animals were sacrificed. Also in this case, the beneficial effect of MSC on Mh T cell infiltration was much reduced in mice receiving 1-DMT compared to controls (Fig. 8, C and D). We therefore conclude that the immunosuppressive effect of MSC requires the presence of recipient phagocytic cells or IDO production.

### Example 9

PBMC from inflammatory bowel disease (IBD) patients (IBD, n=82) or healthy controls (HC, n=8) were co-cultured with MSC for 4 hours at a 20:1 PBMC:MSC ratio and apoptosis assessed by Annexin-V/7-AAD staining by flow cytometry. The results are shown in Figure 16.

It is clear that in patients affected by IBD, there is a cohort of high and low killers. Similarly to what was observed for GvHD patients, the cut-off of 13% seems to distinguish the 2 cohorts of patients.

Further investigations into the molecular basis of the difference were carried out.

In particular MSC (5 x 10⁴) and patients PBMC (1 x 10⁶) were co-cultured for 24 hours, either with or without addition of a pan-caspase inhibibtor, Z-VAD-FMK (50µM). Supernatant was collected and PGE2 levels were measured by ELISA. Results are shown in Figure 17.

These results show that after physical contact with patients' PBMC, MSC are induced to secrete high levels of prostaglandin E2 (PGE2). The induction of PGE2 is entirely dependent on caspase, because the addition of a caspase inhibitor in culture largely reduce PGE2 levels (Figure 17A). Thus, this indicates that the activity correlates with the ability of PBMC to induce MSC apoptosis. The levels showed good correlation with the induction of annexinV+ cells in those samples (see Figure 17B).

Thus PGE2 provides a good marker for the apoptosis inducing activity of PBMCs to MSCs which may be used as an alternative to annexinV+ cell detection. This marker has the advantage that it can be measured more simply and objectively by an independent operator, using for example a simple ELISA method.

### Materials and Methods

This study aimed to verify whether MSC undergo apoptosis after infusion and to test the role played by MSC apoptosis in the initiation of recipient-derived tolerogenic immune response.

A mouse model of GvHD, in which the disease is mediated by the expansion and activation of Mh CD8+ cells in the recipient, was chosen for three important reasons: it recapitulates a minor mismatch between donor and recipient; T cells effecting GvHD can be precisely enumerated; there is proof-of-principle that MSC are effective in treating GvHD. Furthermore, human MSC were used in order to avoid the confounding effects of recipient cytokines on MSC immune-modulating function. In this system, murine inflammatory cytokines will not cross-react with the corresponding human receptors and will not activate immunosuppressive molecules in human MSC, whilst retaining the ability to expand murine effector cells mediating GvHD. Depletion of phagocytes and inhibition of IDO production were conceived as loss of function experiments to assess the requirement of these factors in the delivery of MSC apoptosis-dependent immunosuppression.

A mouse model of ovalbumin (OVA)-induced allergic airway inflammation was used to assess whether the causative relationship between cytotoxic cells and MSC apoptosis in the delivery of MSC immunosuppression is valid in a disease associated with Th2-type inflammation.

No randomization method was used. In all experiments, animals were randomly allocated to control or experimental groups. No blinding approach was adopted. No statistical method was used to predetermine sample size, which was estimated only on previous experience with assay sensitivity and the different animal models. Unless otherwise specified, three independent experimental replicates were performed.

To demonstrate that the presence of cytotoxic cells against MSC in GvHD patients could be predictive of MSC therapeutic activity, samples from GvHD patients were collected and tested for their ability to induce MSC apoptosis in a cytotoxic assay within 24 hours before MSC infusion. At the time of performing the assay and cytofluorimetric analysis, the operator was blind to patients' clinical details. All patients were affected by steroid-resistant GvHD and received MSC for compassionate use. PBMC from healthy donors were used as controls. All samples were collected after informed consent was obtained in accordance with the local ethics committee requirements.

### MSC preparations

Clinical grade BM-derived human MSC were generated from BM aspirates collected from the iliac crest of healthy donors. Briefly, 2 ml of BM aspirate were collected in a tube with 100 µl preservative-free heparin. The cells were plated within 24 hours at a density of 10-25 million/636 cm2 by using alpha modified Eagle's medium (ThermoFisher Scientific, Paisley, UK), conservative-free heparin (1 UI/ml) (Wockhardt UK Limited, Wrexham, UK) and 5% platelet lysate and then incubated for 3 days at 37 °C and 5% CO2 ambience. Non-adherent cells were discarded using phosphate buffered saline (ThermoFisher Scientific, Paisley, UK). When cell confluence of 90-100% was achieved cells were detached with Trypsin-EDTA (0.05% trypsin, 0.5γ mM EDTA•4Na) (ThermoFisher Scientific, Paisley, UK) and reseeded at a density of 5000 cells/cm2. MSC were used at passage 2 for all in vivo experiments, whilst they were used by passage 8 for the in vitro experiments. In the latter case we did not observe any difference in terms of apoptosis susceptibility between different passages. Released criteria were based on positivity (>80%) for CD105, CD90, CD73, negativity (<2%) for CD3, CD14, CD19, CD31, CD45.

### Mice and disease models

No randomization method was used. In all experiments animals were randomly allocated to control or experimental groups. No blinding approach was adopted.

### Cell preparations and media

Cultures were carried out in complete RPMI 1640 medium containing GlutaMAXTM,HEPES (25mM), Penicillin 5000 U/ml and Streptomycin 5000 µg/ml(ThermoFisher Scientific, Paisley, UK), foetal bovine serum 10% (Labtech.com,Uckfield, UK).

Human peripheral blood samples from healthy donors were procured by the National Blood Service (Colindale, UK) as leukocyte cones. Samples from GvHD patients were collected within 24 hours before MSC injection. PBMC were isolated by density gradient separation on Histopaque-1077 (Sigma-Aldrich Company Ltd, Dorset, UK). mSpl were isolated through a cell strainer (BD Falcon, Oxford, UK), whilst lungs were cut into small pieces and incubated with Collagenase type IV (250 U/ml) (Lorne Laboratories, Reading, UK), DNAse I from bovine pancreas (250 U/ml) (Merk Millipor,Watford, UK) and foetal bovine serum 6.25% at 37° C for 1 hour.

### Imaging of MSC

Luc-MSC were transfected with the pGL3-Control vector containing the SV40 promoter for the expression of *Luc*+ (Promega, Southampton, UK) or with pECFPDEVDR-Venus (Addgene, Teddington, UK) using electroporation (Gene Pulser Xcell,BioRad, Kidlington, UK). Cells were suspended in a total volume of 250 µl of buffer and electroporated in 0.4 cm gap cuvettes using 10 µg of DNA at 250 volts and 950 F. When pECFP-DEVDR-Venus was used, the donor fluorophore pECFP and the acceptor Venus-YFP were linked through the flexible linker DEVDR which is recognized and cleaved by the active form of caspase 3. In this system caspase 3 activity can be monitored through the analysis of the Forster Resonance Energy Transfer (FRET) between pECFP and Venus-YFP. When caspase 3 is not active, the flexible linker DEVDR remains intact and energy transfer from pECFP is allowed with emission of YFP signal. Conversely, in the presence of caspase 3 activation DEVDR is cleaved, thus energy transfer is lost and the pECFP signal increases.

For confocal imaging, pECFP-DEVDR-Venus transfected MSC were plated in complete RPMI at a concentration of 1x10⁵ cells in a 30 mm x 10 mm dish (Corning, Flintshire, UK) and let adhere overnight. The following day PHA-aPBMC were added at a PBMC:MSC ratio of 40/1. Where indicated, pan caspase inhibitor Z-VAD-FMK (50 µM), perforin inhibitor EGTA (4 mM), GrB inhibitor Z-AAD-CMK (300 µM) were used.

Living cell imaging was acquired every 3 minutes for 180 minutes using a Leica TCS SP5 II Confocal Microscope, with 488 nm and 407 nm lasers. The images were processed and analyzed by using the software "R" and EBImage package. *In vivo* imaging was performed injecting i.v. 1x10⁶ luc-MSC into naive C57BL/6, BM or GvHD mice 3 days after the transplant in the GvHD model. In the airway inflammation model, luc-MSC were infused i.v. in naïve Balb/C or OVA-treated mice 1 hour after the last OVA challenge. After one additional hour, mice were anesthetized with isoflurane (1.5% isofluorane, 98.5% Oxiygen), injected i.p. with 3 mg of VivoGloTM Casp 3/7 Substrate Z-DEVD Aminoluciferine (Promega, Southampton, UK) and imaged using IVIS® Lumina III (PerkinElmer, Waltham, USA) system for a total time of 5 minutes. Images were analyzed by using the software "R" and EBImage package to obtain mean TLS. Confirmation of the presence of transfected MSC was obtained injecting mice with VivoGloTM Luciferin (Promega, Southampton, UK).

### Pre-activation of human PBMC and murine CD8+ cells

PHA-aPBMC were obtained plating 5x10⁶ human PBMC in 24-well plate in the presence of PHA (5 µg/ml) (Sigma-Aldrich Company Ltd, Dorset, UK) in a final volume of 2 ml of complete RPMI for 72 hours. MLR-aPBMC were obtained using one-way MLR in which PBMC from one donor (stimulators) were irradiated (30 Gy) and cocultured with the PBMC of an unrelated donor (responder) at a stimulator: responder ratio of 1/1 in complete medium at a density of 0.75x10⁶ cells/cm2. Cells were then incubated at 37° C, 5% CO2 for 5 days. NK cells were purified by positively selecting CD56+ cells from healthy donor PBMC (Miltenyi Biotec Ltd, Bisley, UK) and activated with recombinant human-IL-2 (1000 U/ml). NY-ESO1-specific CD8+ T cell clone (Clone 4D8) was kindly supplied by Prof. Vincenzo Cerundolo (Institute of Molecular Medicine, Oxford university, UK). The clone was expanded in complete RPMI 1640 with Sodium Pyruvate (1 mM), 2-Mercaptoethanol (0.05 mM) (ThermoFisher Scientific, Paisley, UK), recombinant human-IL-2 (400 U/ml) (Peprotec EC Ltd, London, UK) and PHA (5 µg/ml) (Sigma-Aldrich Company Ltd, Dorset, UK).

Mh CD8+ were stimulated using the following protocol: 5x10⁶ purified CD8+ Mh cells were plated in 24-well plates in the presence of CD3/CD28-coated beads (Dynabeads®) (ThermoFisher Scientific, Paisley, UK) in a final volume of 2 ml of complete RPMI and incubated for 72 hours.

### Immunosuppressive assay

Serial dilutions of human MSC were plated in a flat bottom 96-well plate and let adhere overnight in 100 µl of complete RPMI. Where indicated, MSC cultures were exposed to human Interferon-γ (hIFN-γ) and human TNF-α (hTNF-α), murine IFN-γ (mIFN-γ)and murine TNF-α(mTNF-α) (20 ng/ml each) (all cytokines were from Peprotec EC Ltd, London, UK), supernatant from PHA-aPBMC or from ConA-aSpl. The following day, 5x10⁵ Balb/C mSpl were labelled with Carboxyfluorescein Diacetate Succinimidyl Ester dye (ThermoFisher Scientific, Paisley, UK) and plated with MSC at escalating MSC/mSpl ratios. Culture controls consisted of mSpl plated without MSC in the presence (positive control) or in the absence of ConA (negative control). Proliferation of mSpl was then assessed by flow-cytometry after 72 hours and expressed as the percentage of the proliferation obtained at each MSC/mSpl dilution in comparison with the one obtained in the positive control culture. Results were expressed as percentage of inhibition.

### Cytotoxic Assay

1x10⁵ MSC were plated overnight in a total volume of 500 µl. The day after preactivated immune cells were plated at different concentrations (2.5 to 40/1 effector:MSC ratios). MSC apoptosis was then tested at different time points using flow-cytometry or confocal microscopy analysis. Eventually, the assay was performed for 4 hours in the vast majority of the cases. At flow-cytometry MSC were identified as CD45- cells.

Antigen-specific cytotoxic activity of clone 4D8 was tested using T2 cells pulsed with NY-ESO-1 antigen (epitope SLLMWITQC) at a concentration of 0.1 µM for 1 hour. In the competition assay, T2 (from Hans Stauss, University College London) and K562 cells (from Junia Melo, Imperial College London) were discriminated from effector cells by CellTraceTM Violet labelling. The tracer concentration was optimized for the T2 (1 µM) and K562 (2.5 µM) cells. Cell lines were tested for mycoplasma contamination before use.

When flow-cytometry was used, the level of apoptosis was assessed using the PE annexin-V apoptosis detection kit (BD Biosciences, Oxford, UK). Unless specified, apoptotic cells were identified as annexin-V+/7-AAD- cells.

### Inhibitors

Where indicated, cultures were supplemented with pan-caspase inhibitor Z-VAD-FMK (10 µM in the flow-cytometry experiments or 50 µM in the living cell confocalexperiments) (R&D System, Oxon, UK), perforin inhibitor EGTA (4 mM) (Sigma-Aldrich Company Ltd, Dorset, UK), GrB inhibitor Z-AAD-CMK (300 µM) (Merk Millipor, Watford, UK), neutralizing antibodies against HLA-DR (clone L243) (50 µg/ml), human HLA-A,B,C (clone W6/32) (100 µg/ml) (BD Biosciences, Oxford, UK), TNF-α antagonist Etanercept (Enbrel®) (10 µg/ml or 100 µg/ml) (Amgen, Cambridge, UK). Each reagent was incubated with MSC 1 hour before the culture with effector killer cells. In all cases, the concentration of the corresponding inhibitor was kept for the duration of the cytotoxic assay.

The neutralizing anti-CD178 (Clone NOK-1) (10 µg/ml or 100 µg/ml) (BD Biosciences, Oxford, UK), anti-TRAIL (clone 2E2) (10 µg/ml or 100 µg/ml) (Enzo Life Sciences, Exeter, UK) antibodies, MYR Protein Kinase-Cζ Pseudosubstrate (PKCζ-PS) (10 µM, 25 µM or 75 µM) (ThermoFisher Scientific, Paisley, UK) and Etanercept (10 µg/ml or 100 µg/ml) were incubated with effector killer cells for 2 hours before the cultures with MSC. In all cases, the concentration of the corresponding inhibitor was kept for the duration of the cytotoxic assay.

### Flow-cytometry

The following antibodies specific for murine molecules were used: anti-CD45 (FITC, Clone 30-F11) (eBiosciences Ltd, Hatfield, UK), anti-Vβ8.3 (FITC, Clone 1B3.3), antiCD8 (APC, Clone 53-6.7), antiCD4 (PE, Clone H129.19), anti-CD19 (APC-H7, Clone 1D3), anti-NK1.1 (PerCP-Cy5.5, Clone PK136) (BD Biosciences, Oxford, UK), anti-CD11b (PerCP-Cy5.5, clone M1/70), anti-CD11c (APC-Cy7, clone n418), anti-Ido1 (Alexa Fluo647, clone 2e2) (BioLegend, London, UK). For human specific molecules, the following antibodies were used: anti-CD45 (FITC, clone 2D1), anti-CD8 (APC, Clone SK1), anti-CD4 (PE, Clone SK3), anti-CD11b (PerCP-Cy5.5, clone M1/70), anti-CD56 (FITC, clone HCD56) (BD Biosciences, Oxford, UK). All samples were acquired using BD FACS Canto II using the software FACS Diva and analyzed with Flow-jo software. FRET and Caspase activity (CAf) were assessed by flow-cytometry as previously described (He et al. Am J. Pathol 164, 1901-1913 (2004)).

### Real Time quantitative PCR

MSC RNA was obtained from TRIzol® (ThermoFisher Scientific, Paisley, UK) lysates and extracted using RNeasy Mini Kit (Qiagen, Manchester, UK). Real Time quantitative PCR (qRT-PCR) was performed following TaqMan® RNA-to-CT™ 1-Step Kit instructions (ThermoFisher Scientific, Paisley, UK), using 20 ng of RNA template per reaction. Assays were carried out in duplicates on an StepOnePlus RT PCR system thermal cycler (Applied Biosystem, UK) using TaqMan primers (all purchased from ThermoFisher Scientific, Paisley, UK). The human primers used were the following: IDO2 (Hs01589373_ml), TSG6 (Hs01113602_ml) and PTSG2 (Hs00153133_m1) and HPRT1 (Hs02800695_m1) as housekeeping gene. Data were then analysed using StepOneTM software version 2.1 and relative quantification obtained with ΔΔCt method, considering untreated MSC as reference.

### Statistics

Results were expressed as mean±SD. The unpaired Student *t* test was performed to compare 2 mean values. One-way ANOVA and Tukey's Multiple Comparison test was used to compare 3 or more mean values. Probability of null hypothesis less than 5% (p>.05, two-sided) was considered statistically significant. No statistical methods were used to predetermine sample size, which was estimated only on previous experience with assay sensitivity and the different animal models.

**Table 1. Clinical features of GvHD patients**

| **Diagnosis** | **Donor type** | **GvHD** | **Grade** | **Organs involved** | **Concomitant therapy for GvHD** | **MSC Dose (x10⁶/Kg)** | **Response** |
|---|---|---|---|---|---|---|---|
| DLBCL | SIB | Late onset | 3 | skin, liver | Steroid, MMF, Infliximab | 1.60 | NR |
| CLL | SIB | Late onset | 4 | gut | Steroid, MMF, Infliximab, Alemtuzumab | 2.80 | R |
| HL | VUD | Acute | 3 | skin, Gut | Steroid | 3.00† | NR† |
| | | | | | | 7.40‡ | R‡ |
| CML | VUD | Late onset* | 3 | Skin, Liver | MMF | 3.00 | NR |
| AML | VUD | Chronic* | N/A | Skin | Steroid, CSA | 2.70 | NR |
| AML | SIB | Acute | 3 | Gut | Steroid, CSA | 2.10 | R |
| CML | VUD | Acute* | 4 | Gut | Steroid, CSA | 2.90 | R |
| AML | VUD | Acute | 4 | Skin, gut | Steroid, CSA | 3.10 | NR |
| FL | SIB | Acute | 4 | Skin, gut, Liver | Steroid, CSA, MMF | 1.60 | R |
| MM | SIB | Acute | 4 | Gut | Steroid, Infliximab | 2.10 | NR |
| AML | VUD | Acute | 4 | Gut | Steroids, Budenofalk, CsA | 1.28 | NR |
| pre-B ALL | UUD | Acute | 4 | Skin | Steroids, Topic glucorticoids | 1.03 | NR |
| MDS/RAEB-2 | VUD | Acute | 4 | Gut | Steroids, Tacrolimus,MMF, Etanercept, Ruxolitinib, MTX, Alemtuzumab, CsA | 1.55 | NR |
| Mixed AML/T-ALL | VUD | Acute | 4 | Gut | Steroids, CSA | 1.33 | NR |
| MM | VUD | Acute | 3 | Gut | Steroids, CSA | 1.01 | NR |
| B-ALL, BCRABL⁺ | SIB | Acute | 3 | Skin, Liver | Steroids, ECP, CsA | 1.11 | NR |

| | | | | | | | |
|---|---|---|---|---|---|---|---|
| ^{†}: first dose ^{‡}: second dose *: GvHD post-Donor Lymphocyte Infusion AML: Acute Myeloid Leukemia; CML: Chronic Myeloid Leukemia; CLL: Chronic Lymphocytic Leukemia; CSA: Cyclosporine; DLBCL: Diffuse Large B-Cell Lymphoma; FL: Follicular Lymphoma; HL: Hodgkin Lymphoma; NR: no response; MM: Multiple Myeloma; MMF: Mycophenolate Mofetil; R: response; SIB: HLA-identical sibling; VUD: Volunteer Unrelated Donor. | | | | | | | |

The invention is now described by way of numbered paragraphs.
Paragraph 1. A method for identifying a patient likely to respond to immunosuppressive treatment using live mesenchymal stromal cells (MSCs), said method comprising determining whether a sample from said patient is able to induce at least some apoptosis to occur in live MSCs *in vitro,* and where the ability of the sample to induce said apoptosis is indicative of responsiveness of said patient to said immunosuppressive treatment.
Paragraph 2. A method according to paragraph 1 wherein the ability of the sample to cause apoptosis in MSCs is determined by incubating said sample with live MSCs and thereafter, detecting the presence of apoptotic MSCs in the incubate.
Paragraph 3. A method according to any one of the preceding paragraphs wherein a finding that at least 11% of MSCs added to the sample are apopotic after incubation is indicative of responsiveness of said patient to said immunosuppressive treatment.
Paragraph 4. A method according to paragraph 1 wherein the ability of the sample to cause apoptosis in MSCs is determined by incubating said sample with live MSCs, detecting the level of prostaglandin E2 (PGE2) in the incubate or a supernatant obtained therefrom, and relating the level to the responsiveness of said patient to said immunosuppressive treatment.
Paragraph 5. A method according to paragraph 5 wherein a level of PGE2 in excess of 4000pg/ml is indicative of responsiveness of said patient to said immunosuppressive treatment.
Paragraph 6. A method according to paragraph 4 or paragraph 5 wherein the level of PGE2 is determined using a quantitative ELISA.
Paragraph 7. A method according to any one of the preceding paragraphs, wherein a patient determined to be responsive to immunosuppressive treatment using live MSCs is prescribed for treatment with MSCs, and wherein a patient determined to be non-responsive to said immunosuppressive treatment is prescribed treatment with apoptotic MSCs.
Paragraph 8. A method of using live mesenchymal stromal cells (MSCs)in vitro, the method comprising contacting the live MSCs in vitro with a biological sample from a patient, and detecting either the presence of apoptotic MSCs among the live MSCs in vitro or elevated levels of prostaglandin E2 (PGE2) in the sample, wherein the presence of apoptotic MSCs among the live MSCs or elevated levels of PGE2 in vitro is indicative of responsiveness of the patient to an immunosuppressive treatment using live MSCs.
Paragraph 9. A method according to paragraph 8, further comprising contacting a plurality of samples of live MSCs *in vitro,* with a biological sample from a different patient in a plurality of patients, and detecting the presence of apoptotic MSCs in each sample of live MSCs *in vitro.*
Paragraph 10. A method according to paragraph 8 or paragraph 9 wherein the or each contacting step comprises incubating the biological sample with live MSCs, and wherein the or each detecting step comprises detecting the presence and/or determining the potency of apoptotic MSCs in the incubate.
Paragraph 11. A method according to any one of paragraphs 8 to 10 wherein a finding that at least 11% of the live MSCs in vitro are apopotic after contact with the sample, or that the level of prostaglandin E2 in a supernatant is in excess of 4000pg/ml is indicative of responsiveness of the patient to the immunosuppressive treatment.
Paragraph 12. A method according to any one of paragraphs 8 to 11, wherein if the presence of apoptotic MSCs among the live MSCs and/or elevated levels of PGE2 *in vitro* is detected, the patient is treated with live MSCs, and wherein if the presence of apoptotic MSCs among the live MSCs *in vitro* is not detected, the patient is treated with apoptotic MSCs.
Paragraph 13. A method according to any one of the preceding paragraphs wherein the sample is a blood or serum sample.
Paragraph 14. A method according to any one of the preceding paragraphs wherein the presence of apoptotic MSCs is determined by confocal microscopy or flow cytometry.
Paragraph 15. A method of stratifying patients for treatment with MSC immunosuppression therapy, said method comprising carrying out a method according to any one of paragraphs 1 to 14, identifying patients likely to respond to immunosuppressive treatment using live mesenchymal stromal cells (MSCs) for treatment with live MSCs, and identifying those who are not likely to respond to said immunosuppressive treatment for treatment with apoptotic MSCs.
Paragraph 16. A method for identifying a subject's fitness to recover from tissue injury, said method comprising determining whether a sample from said subject is able to induce at least some apoptosis to occur in live MSCs *in vitro,* and/or detecting elevated levels of PGE2, and wherein the ability of the sample to induce said apoptosis, or the presence of elevated levels of PGE2, is indicative of said patient's capacity to recover.
Paragraph 17. A method for producing an immunosuppressive effect in a patient in need thereof, said method comprising determining whether said patient is likely to respond to immunosuppressive treatment using live mesenchymal stromal cells (MSCs), by detecting the presence of at least some apoptosis in live MSCs *in vitro,* in contact with a biological sample from the patient, administering to the patient an effective amount of live MSCs when the presence of apoptosis in the live MSCs *in vitro* is detected, and administering to the patient an effective amount of apoptotic MSCs when the presence of apoptosis in the live MSCs *in vitro* is not detected.
Paragraph 18. A method according to paragraph 17 wherein the patient is suffering from an immune-mediated disease or condition or requires regenerative medicine to stimulate tissue repair.
Paragraph 19. A method according to paragraph 17 or paragraph 18 wherein the disease or condition is allo-immune or autoimmune disease, or is for the prevention or treatment of rejection of a transplanted organ.
Paragraph 20. A method according to paragraph 19 wherein the disease is Graft-vs-Host-Disease, multiple sclerosis, inflammatory bowel diseases such as Crohn's disease or ulcerative colitis, arthritis such as rheumatoid arthritis, ankylosing spondylitis or psoriatic arthritis, and acute respiratory distress syndrome.
Paragraph 21. A method according to any one of paragraphs 15 to 20 wherein efficacy of the treatment in the patient is monitored by detecting the presence of phagocytes or IDO in a sample taken from the patient after administration of MSC.
Paragraph 22. A kit comprising components required to carry out a method according to any one of paragraphs 1 to 12.
Paragraph 23. Apoptotic MSCs for use in the treatment of immune-mediated disease or conditions or in regenerative medicine to stimulate tissue repair.
Paragraph 24. Apoptotic MSCs for use in a method according to any one of paragraphs 7, 12, 15, 17-20.
Paragraph 25. Apoptotic MSCs according to paragraph 23 or 24 wherein the disease is allo-immune or autoimmune disease, or is for the prevention or treatment of rejection of a transplanted organ.
Paragraph 26. Apoptotic MSCs according to paragraph 25 wherein the disease is Graft-vs-Host-Disease, multiple sclerosis, inflammatory bowel diseases such as Crohn's disease or ulcerative colitis, arthritis such as rheumatoid arthritis, ankylosing spondylitis or psoriatic arthritis, and acute respiratory distress syndrome.
Paragraph 27. A method for producing apoptotic MSCs for use in immunosuppression therapy, said method comprising incubating live MSCs with a pharmaceutically acceptable apoptosis inducing agent for a period sufficient to form a cell culture in which at least 30% of the MSCs are apoptotic within a period of less than 24 hours.
Paragraph 28. A method according to paragraph 27 wherein pharmaceutically acceptable apoptosis inducing agent is a biological agent.
Paragraph 29. A method according to paragraph 28 wherein said biological agent is a serine protease and/or an anti-FAS antibody.
Paragraph 30. A method according to paragraph 29 wherein said serine protease is human Granzyme B (GrB).
Paragraph 31. A method according to any one of paragraphs 27 to 30 wherein the pharmaceutically acceptable apoptosis inducing agent comprises a combination of GrB and anti-Fas antibody.
Paragraph 32. Apoptotic MSCs obtainable using a method according to any one of paragraphs 27 to 31.
Paragraph 33. Apoptotic MSCs according to paragraph 32 for use in immunosuppression therapy.
Paragraph 34. Apoptotic MScs according to paragraph 32 or 33 comprising a suicide mechanism, optionally in the form of a suicide gene, particularly a caspase-inducible gene, such as caspase 9 or caspase 3.
Paragraph 35. A pharmaceutical composition comprising apoptotic MSCs according to any one of paragraphs 31 to 33.
Paragraph 36. A composition according to paragraph 35 which further comprises live MSCs.
Paragraph 37. A method for producing an immunosuppressive effect in a patient in need thereof, administering to said patient an effective amount of apoptotic MSCs.
Paragraph 38. A method according to paragraph 37 wherein the patient is suffering from an immune-mediated disease or condition or requires regenerative medicine to stimulate tissue repair.
Paragraph 39. A method according to paragraph 37 or paragraph 38 wherein the disease or condition is allo-immune or autoimmune disease, or is for the prevention or treatment of rejection of a transplanted organ.
Paragraph 40. A method according to paragraph 39 wherein the disease is Graft-vs-Host-Disease, multiple sclerosis, inflammatory bowel diseases such as Crohn's disease or ulcerative colitis, arthritis such as rheumatoid arthritis, ankylosing spondylitis or psoriatic arthritis, and acute respiratory distress syndrome.
Paragraph 41. A method according to any one of paragraphs 37 to 40 wherein efficacy of the treatment in the patient is monitored by detecting the presence of phagocytes or IDO in a sample taken from the patient after administration of MSC.
Paragraph 42. Use of prostaglandin E2 (PGE2) and/or use of apoptotic MSCs as biomarkers for determining fitness to recover in a patient and/or responsiveness to immunosuppressive treatment.
Paragraph 43. Use according to paragraph 42, comprising detection of the presence and amount of apoptotic MSCs and/or detecting elevated levels of PGE2 in a sample from said patient.

## Claims

1. Apoptotic MSCs for use in the treatment of immune-mediated disease or conditions or in regenerative medicine to stimulate tissue repair.

2. Apoptotic MSCs according to claim 1 wherein the disease is allo-immune or autoimmune disease, or is for the prevention or treatment of rejection of a transplanted organ.

3. Apoptotic MSCs according to claim 1 wherein the disease is Graft-vs-Host-Disease, multiple sclerosis, inflammatory bowel diseases such as Crohn's disease or ulcerative colitis, arthritis such as rheumatoid arthritis, ankylosing spondylitis or psoriatic arthritis, and acute respiratory distress syndrome.

4. A method for producing apoptotic MSCs for use in immunosuppression therapy, said method comprising incubating live MSCs with a pharmaceutically acceptable apoptosis inducing agent for a period sufficient to form a cell culture in which at least 30% of the MSCs are apoptotic within a period of less than 24 hours.

5. A method according to claim 4 pharmaceutically acceptable apoptosis inducing agent is a biological agent, and wherein said biological agent is a serine protease and/or an anti-FAS antibody.

6. A method according to claim 5 wherein said serine protease is human Granzyme B (GrB).

7. A method according to any one of claims 4 to 6 wherein the pharmaceutically acceptable apoptosis inducing agent comprises a combination of GrB and anti-Fas antibody.

8. Apoptotic MSCs obtainable using a method according to any one of claims 4 to 7 for use in immunosuppression therapy.

9. Apoptotic MScs according to claim 8 comprising a suicide mechanism, optionally in the form of a suicide gene, particularly a caspase-inducible gene, such as caspase 9 or caspase 3.

10. A pharmaceutical composition comprising apoptotic MSCs according to any one of claims 8 or 9, which further comprises live MSCs.

11. Use of prostaglandin E2 (PGE2) and/or use of apoptotic MSCs as biomarkers for determining fitness to recover in a patient and/or responsiveness to immunosuppressive treatment.

12. Use according to claim 11, comprising detection of the presence and amount of apoptotic MSCs and/or detecting elevated levels of PGE2 in a sample from said patient.

13. A method of identification of suitably potent MSCs that can best be induced to undergo apoptosis and/or to produce PGE2, comprising
contacting the live MSCs in vitro with a biological sample from a patient, and detecting either the presence of apoptotic MSCs among the live MSCs in vitro or elevated levels of prostaglandin E2 (PGE2) in the sample, wherein the presence of apoptotic MSCs among the live MSCs or elevated levels of PGE2 in vitro is indicative of potency.

14. A method of using live mesenchymal stromal cells (MSCs) in vitro, the method comprising
using a plurality of samples of live MSCs in vitro, each of which is contacted with biological samples from different patients in a plurality of patients, and the presence of apoptotic MSCs, or of elevated levels of PGE2, in each sample of live MSCs in vitro is detected, thereby identifying suitably potent MSCs.

15. A method according to claim 13 or claim 14 wherein the or each contacting step comprises incubating the biological sample with live MSCs, and wherein the or each detecting step comprises detecting the presence and/or determining the potency of apoptotic MSCs in the incubate.
